# EUROPEAN PATENT SPECIFICATION

(11) **EP 4 121 278 B1**
(45) Date of publication and mention of the grant of the patent: **26.11.2025**
(21) Application number: 21771661.2
(22) Date of filing: 16.03.2021
(51) Int. Cl.: B29C 65/18, B29C 65/74, B29C 65/78, A61M 39/18, B29C 65/20, B29C 65/24, B29L 23/00

(54) **STERILE TUBE CONNECTING DEVICE**
STERILE ROHRVERBINDUNGSVORRICHTUNG
DISPOSITIF DE RACCORDEMENT DE TUBE STÉRILE

(30) Priority: 17.03.2020 SG 10202002436X
(43) Date of publication of application: 25.01.2023
(73) Proprietor: GenesisBPS, Ramsey, NJ 07446 (US)
(72) Inventor: KOH, Kee Joo, Willy, Singapore 486772 (SG); LEE, Chia Ta, Singapore 486772 (SG); GOH, Puay Hian, Singapore 486772 (SG); FOO, Biao Jin Ryan, Singapore 486772 (SG)
(74) Representative: Bardehle Pagenberg Partnerschaft mbB Patentanwälte Rechtsanwälte
(86) International application number: PCT/SG2021/050138
(87) International publication number: WO 2021/188049

(56) References cited:
- WO-A1-96/26814
- WO-A2-92/05945
- JP-B2- 3 220 229
- US-A- 4 610 670
- US-A- 4 610 670
- US-A- 4 633 063
- US-A1- 2006 005 371
- US-A1- 2016 250 806

## Description

### FIELD

This invention relates to a sterile tube connecting device for joining two lengths of sterile tubing.

### BACKGROUND

Sterile tube connecting devices are used to join two lengths of sterile tubing together to form a single length of sterile tubing. This involves first cutting the two tubings with a cutting wafer that has been heated to a suitable temperature so that the melted cut ends of both tubings can be brought into contact with each other to form a weld that joins the cut tubings as a single length of tubing. As both tubings must be cut at the same time or the difference in temperature and melting state will cause a weak or uneven weld, leading to a failed connection, during cutting, the wafer should simultaneously cut across both tubings, in a first direction. After cutting, the retained lengths of one of the tubings must be moved relative to the retained length of the other tubing in a second direction in order to axially align the central longitudinal axes of the retained lengths of both tubings. When the retained lengths of both tubings have been axially aligned, the cut surfaces of the retained lengths must then brought towards each other in a third direction that is perpendicular to both the first direction and the second direction in order to bring the melted cut ends of both tubings into contact with each other to form the weld. The mechanisms required to automate the entire cutting, aligning and contacting/welding process involve various different directions of motion of the wafer and the tubings, and is usually achieved using a number of synchronized motors. Existing sterile tube connecting devices are therefore usually bulky and not readily moved for portable on-site use.

Temperature control of the wafer is also crucial in cutting and welding the tubings as thermoplastic tubing are highly heat sensitive. A slight deviation in temperature will cause the tube connection to be either too weak or the weld surface to close off, leading to a failed connection. The distance between the thermoplastic tubings and heating elements used to heat the wafer is also a factor because radiating heat from the heating elements could affect the tubings if the tubings and the heating elements are placed too close together, while heat would be lost from the wafer as it travels between the heating elements and the tubings if they are placed too far apart. Thus, temperature control is difficult to achieve because of the delay of the mechanical transition and varying ambient temperature of the tube connection device during use, leading to inconsistent heat loss.

US 4,610,670 A discloses a process, apparatus and system for making a sterile connection between thermoplastic resin tubes is disclosed. A section of each tube is flattened and a hot cutting means is urged through the flattened sections so as to seal temporarily each tube and to provide molten tube ends. The tubes are aligned with each other and then the desired molten tube ends are urged together to form a joint between the tubes for each pair of tube ends to be connected. Each joint is cooled and then subjected to light stress to open the temporary seal in each tube, thereby providing fluid communication between the joined tubes.

Further prior art is known from JP 3220229 B2, US 4 633 063 A and WO 96/26814 A1.

It is therefore desirable to provide a sterile tube connecting device that is both compact and portable while minimizing heat loss to achieve good welds of the cut tubings.

### SUMMARY

The underlying problem of the present invention is solved by the subject matter of the independent claims.

### BRIEF DESCRIPTION OF FIGURES

In order that the invention may be fully understood and readily put into practical effect, there shall now be described by way of non-limitative example only exemplary embodiments of the present invention, the description being with reference to the accompanying illustrative drawings.
- FIG. 1 A: is a right perspective view of an exemplary sterile tube connecting device.
- FIG. IB: is a left perspective view of the device of FIG. 1.
- FIG. 2 A: is a right perspective view of the device of FIG. 1A with right side features hidden to show more details of left side features.
- FIG. 2B: is a left perspective view of the device of FIG. 1A with left side features hidden to show more details of right side features.
- FIG. 3 A: is a right perspective view of a carriage of the device of FIG. 1.
- FIG. 3B: is a right perspective exploded assembly view of the carriage of FIG. 3 A.
- FIG. 4A: is a right perspective view of a cutting wafer for use with the device of FIG. 1A.
- FIG. 4B: is a right elevation view of the wafer of FIG. 4A.
- FIG. 5 A: is a right elevation view of the device of FIG. 1A where the carriage is moved backward to a loading position.
- FIG. 5B: is a close-up top plan view of a loading mechanism of the device pushing a wafer from a wafer cartridge onto the carriage.
- FIG. 5C: is a right elevation view of the device of FIG. 1A where the carriage is moved forward from the loading position to a heating position.
- FIG. 6 A: is a right elevation view of the device of FIG. 1A where the carriage is moved forward from the heating position towards two tubings (not shown) clamped on the device for cutting and joining.
- FIG. 6B: is a close-up right elevation view of positions of the carriage and wafer, a left tube clamp, and a displacement lock mechanism of the device at the position of FIG. 6A.
- FIG. 6C: is a close-up top plan view of positions of the carriage, the two tubings, the left tube clamp, a right tube clamp, a displacement mechanism assembly, and a welding mechanism assembly of the device at the position of FIG. 6A.
- FIG. 7A: is a right elevation view of the device of FIG. 1A where the carriage is moved forward and upward for the wafer to cut the two tubings (not shown) clamped on the device.
- FIG. 7B: is a close-up right elevation view of positions of the carriage and wafer, the left tube clamp, and the displacement lock mechanism of the device at the position of FIG. 7A.
- FIG. 7C: is a close-up top plan view of positions of the carriage, the two tubings, the left tube clamp, the right tube clamp, the displacement mechanism assembly, and the welding mechanism assembly of the device at the position of FIG. 7A.
- FIG. 8 A: is a right elevation view of the device of FIG. 1A where the carriage is moved forward immediately after the two tubings (not shown) clamped on the device have been cut by the wafer.
- FIG. 8B: is a close-up right elevation view of positions of the carriage and wafer, the left tube clamp, and the displacement lock mechanism of the device at the position of FIG. 8A.
- FIG. 8C: is a close-up top plan view of positions of the carriage, the two tubings, the left tube clamp, the right tube clamp, the displacement mechanism assembly and the welding mechanism assembly of the device at the position of FIG. 8 A.
- FIG. 9A: is a close-up top plan view of positions of the carriage, the two tubings, the left tube clamp, the right tube clamp, the displacement mechanism assembly and the welding mechanism assembly of the device when the carriage is moved forward and downward while the wafer is still in contact with the two cut tubings.
- FIG. 9B: is a right elevation view of the device of FIG. 1A where the carriage is moved forward and downward after the two tubings (not shown) clamped on the device have been cut by the wafer and the wafer is no longer in contact with the two cut tubings.
- FIG. 9C: is a close-up right elevation view of positions of the carriage and wafer, the left tube clamp, and the displacement lock mechanism of the device at the position of FIG. 9B.
- FIG. 10A: is a right elevation view of the device of FIG. 1A where the carriage is moved forward to an extraction position.
- FIG. 10B: is a close-up top plan view of an extraction mechanism engaging the wafer as the carriage is moved forward to the position of FIG. 10A.
- FIG. IOC: is a right elevation view of the device of FIG. 1A where the carriage is moved backward from the extraction position toward the heating position after the wafer is extracted from the carriage by the extraction mechanism.

### DETAILED DESCRIPTION

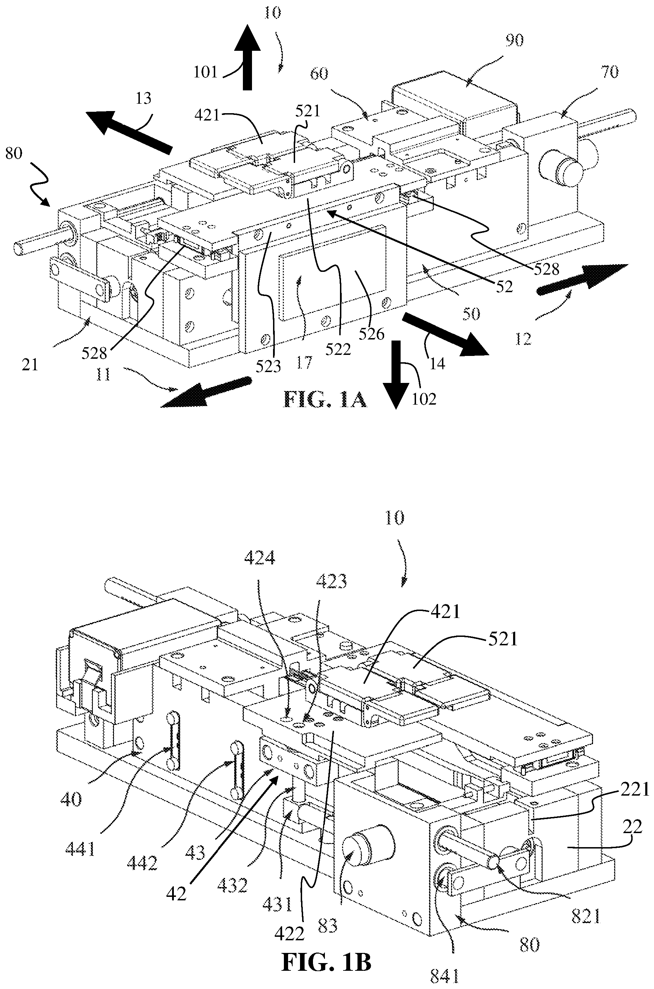

Exemplary embodiments of a sterile tube connecting device 10 and a method 900 of sterile tube connecting will be described below with reference to FIGS. 1A to IOC. The same reference numerals are used across the figures to denote the same or similar parts.

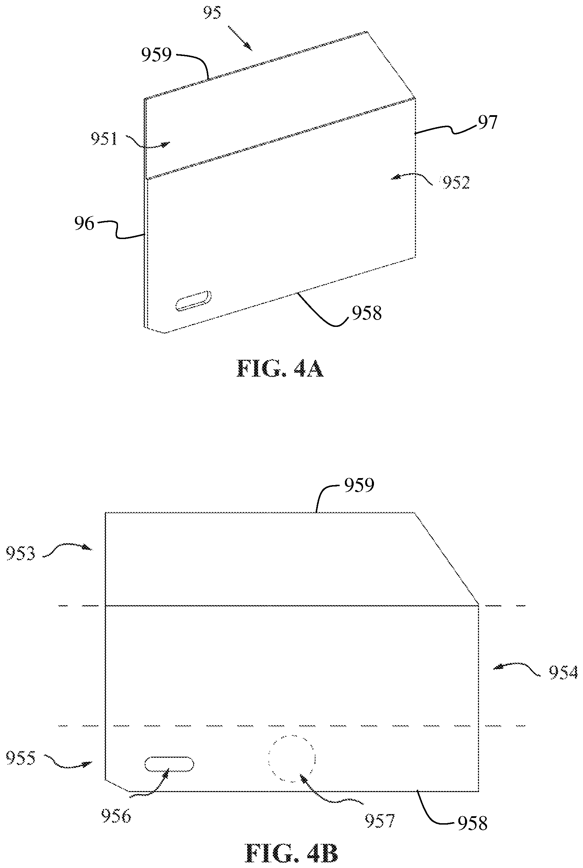

As shown in FIGS. 1A to 2B, in general, the sterile tube connecting device 10 comprises at least an electrical control circuit board 17, a carriage 30 for supporting a cutting blade or wafer 95 thereon, a first tube clamp 421, a second tube clamp 521, a lead screw 26 fixedly connected to the carriage 30, a motor 27 to drive the lead screw 26, and heating elements 611 to heat the wafer 95. The device 10 may also compromise a loading mechanism 70, an extraction mechanism 80 and a cartridge assembly 90 (or wafer cartridge 90). The wafer cartridge 90 is designed to contain multiple wafers 95 as shown in FIG. 4A for use in the device 10. In an exemplary embodiment, the first and second tube clamps 421, 521 are provided as left and right tube clamps 421, 521 respectively, and in the following description will be referred to as such, although in other embodiments, left and right may be interchanged without departing from the spirit of the invention.

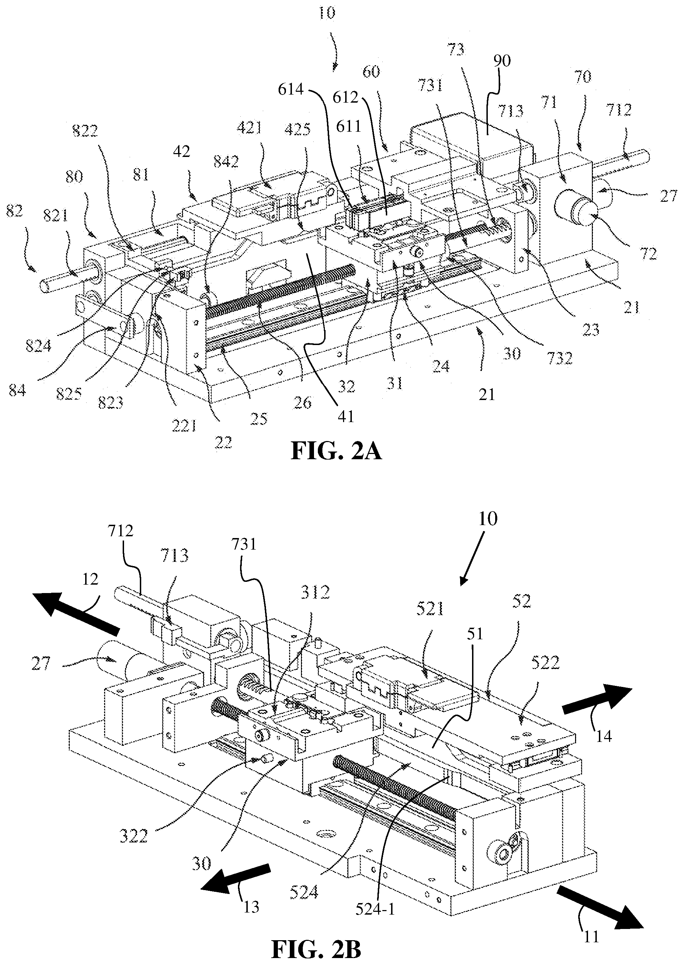

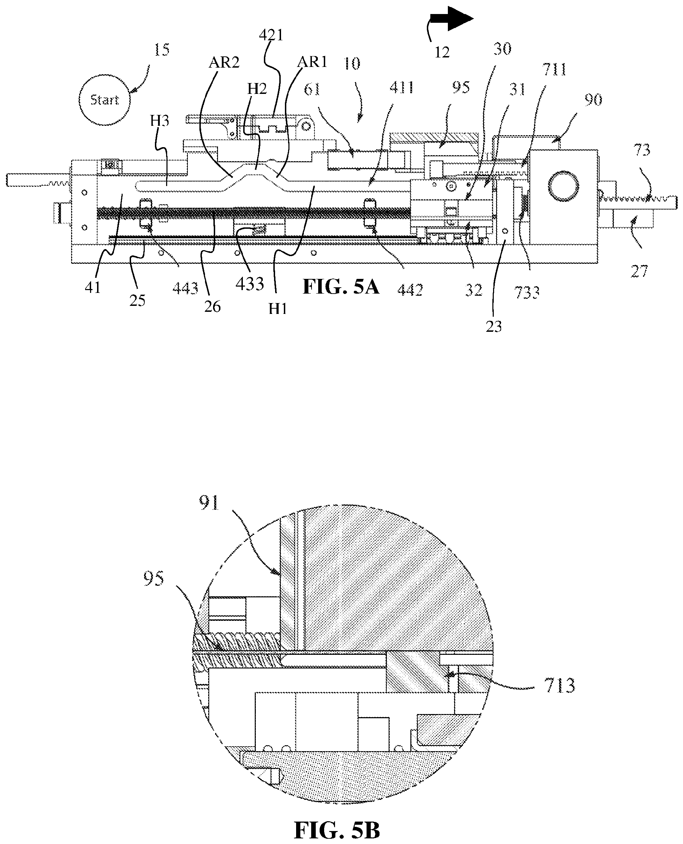

The device 10 may further comprise an upstanding first cam plate 41 (FIG. 2A) and an upstanding second cam plate 51 (FIG. 2B) provided one on each side of a path of travel of the carriage 30 respectively. In the exemplary embodiment, the first and second cam plates 41, 51 are provided as left and right cam plates 41, 51 respectively, and in the following description will be referred to as such, although in other embodiments, left and right may be interchangeable without departing from the spirit of the invention. The left and right cam plates 41, 51 each have a cam track 411, 511 facing the path of travel. The carriage 30 is configured to support and move the cutting wafer 95 in forward 11 and backward 12 longitudinal directions (as indicated in FIG. 1A) and also upward 101 and downward 102 vertical (up and down) directions (as indicated in FIG. 1A) in order to move the wafer 95 to a heating area for heating, move the wafer through two tubings 131, 132 clamped on the device 10 by the left tube clamp 421 and right tube clamp 21 to simultaneously cut both tubings 131, 132 between the left tube clamp 421 and right tube clamp 21, and to return the carriage 30 to a start position, as can be seen in FIGS. 5B, 5C, 6A, 7A, 8A, 9A, 10B and IOC. The carriage 30 is movable in the forward 11 and backward 12 longitudinal directions and at least part of the carriage 30 is also moveable in upward 101 and downward 102 vertical directions as guided by the left and right cam plates 41, 51, as will be described further below. Movement of the carriage 30 is driven by means of the lead screw 26 rotated by the motor 27.

The device 10 and its components such as a displacement mechanism assembly 42 on which the left tube clamp 421 is provided, a welding mechanism assembly 52 on which the right tube clamp 521 is provided, the left tube clamp 421, the right tube clamp 521, the carriage 30, the left cam plate 41, the right cam plate 51, the lead screw 27, the loading mechanism 70 and the extraction mechanism 80 may be made of one or more metallic materials such as aluminum and/or steel. The cartridge case 91 of the wafer cartridge 90 may be made of a plastics material such as polycarbonate that is preferably transparent or translucent.

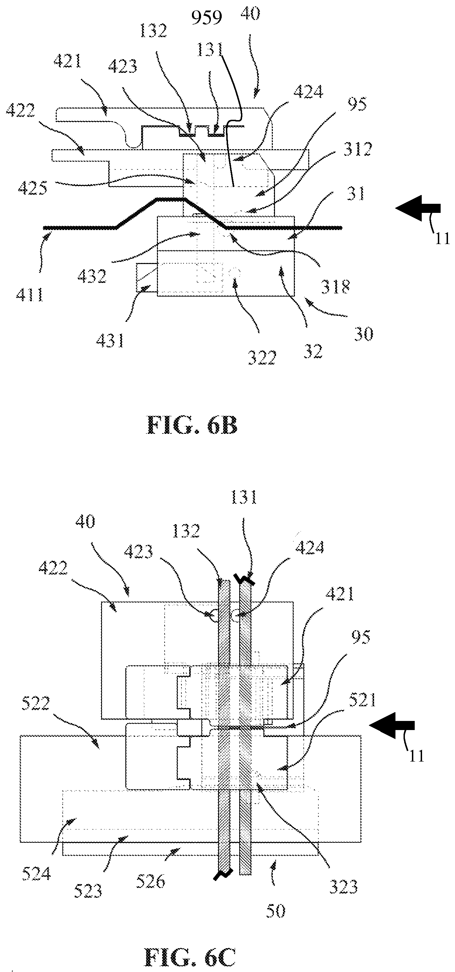

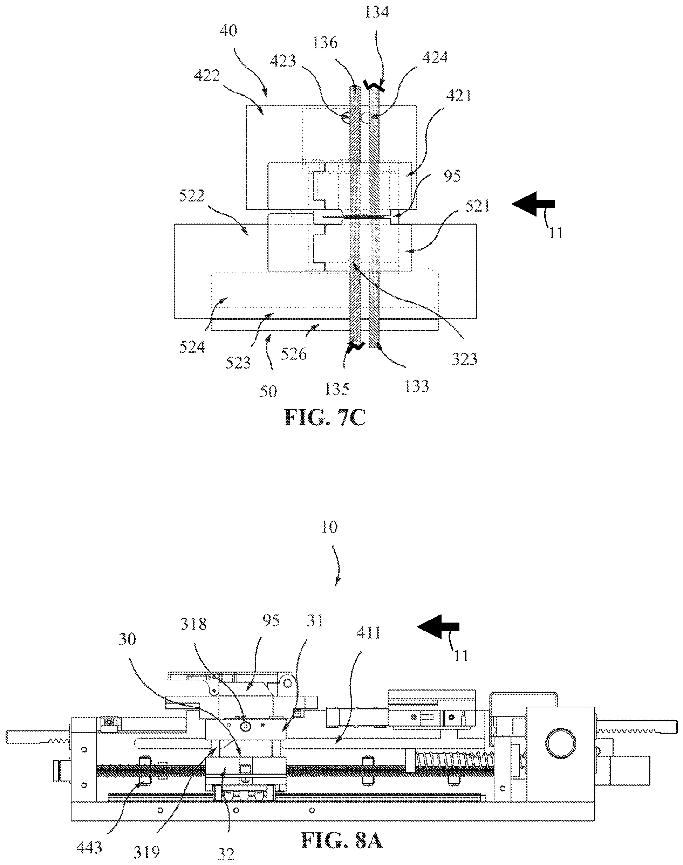

Before and during cutting, both tubings 131, 132 are clamped in place by the left tube clamp 421 and the right tube clamp 421 simultaneously, as can be seen in FIGS. 6C and 7C. The left tube clamp 421 and right tube clamp 521 are provided on left and right sides respectively of the path of movement of the wafer 95 and laterally spaced apart from each other. Both tubings 131, 132 are clamped by the left tube clamp 421 and the right tube clamp 521 such that their central longitudinal axes are parallel with each other. In an exemplary embodiment, the central longitudinal axes of the two tubings 131, 132 when clamped on the device 10 are perpendicular to the longitudinal directions 11, 12, i.e., transversely across the path of movement of the wafer 95. In this way, both tubings 131, 132 can be simultaneously cut between the left tube clamp 421 and right tube clamp 521 as a result of forward 11 and upward 101 movement of the wafer 95 between the left tube clamp 421 and the right tube clamp 521 while keeping the tubing 131, 132 stationary. More details about the wafer cutting movement will be given below.

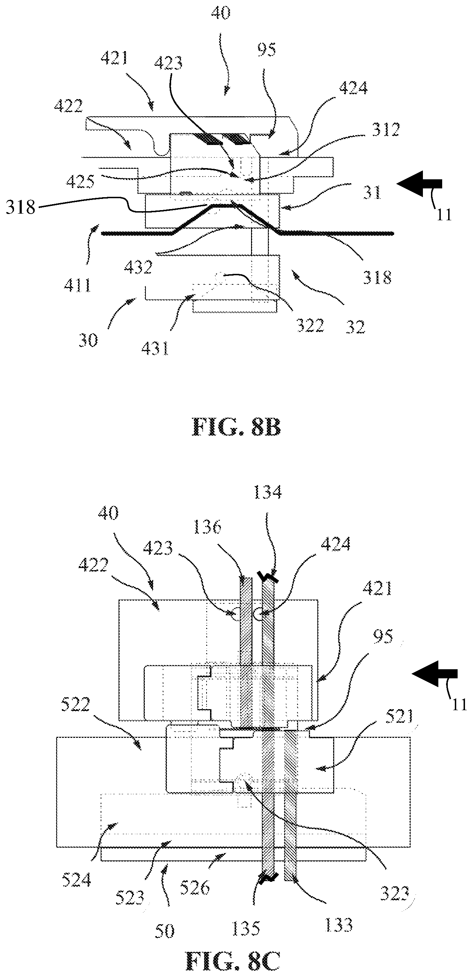

The left tube clamp 421 is provided on top of the displacement mechanism assembly 42 as can be seen more clearly in FIG. IB. The displacement mechanism assembly 42 is longitudinally displaceable in the forward 11 and backward 12 directions in order to longitudinally displace or move the left tube clamp 421 forward 11 after both tubings 131, 132 have been cut by the wafer 95, and to return the left tube clamp 421 backward 12 to a start position. The displacement mechanism assembly 42 may be connected by linear guide rails (not shown) that allow longitudinal movement 11, 12 to an immoveable left upstanding wall 40 (FIG. IB) of the device 10. After both tubings 131, 132 have been simultaneously cut by the heated wafer 95, a retained length 134 of the first tubing 131 remains clamped by the left tube clamp 421 while a retained length 135 of the second tubing 132 remains clamped by the right tube clamp 521. By moving the left tube clamp 421 forward 11, the central longitudinal axes of both retained lengths 134, 135 of the first and second tubings 131, 132 can become axially aligned, as shown in FIG. 8C. The displacement mechanism assembly 42 may further comprise a displacement mechanism clamp mounting plate 422 on which the left tube clamp 421 is fixedly mounted. The displacement mechanism assembly 42 is longitudinally moveable relative to the left cam plate 41. Further description on additional features of the displacement mechanism clamp mounting plate 422 will be given below.

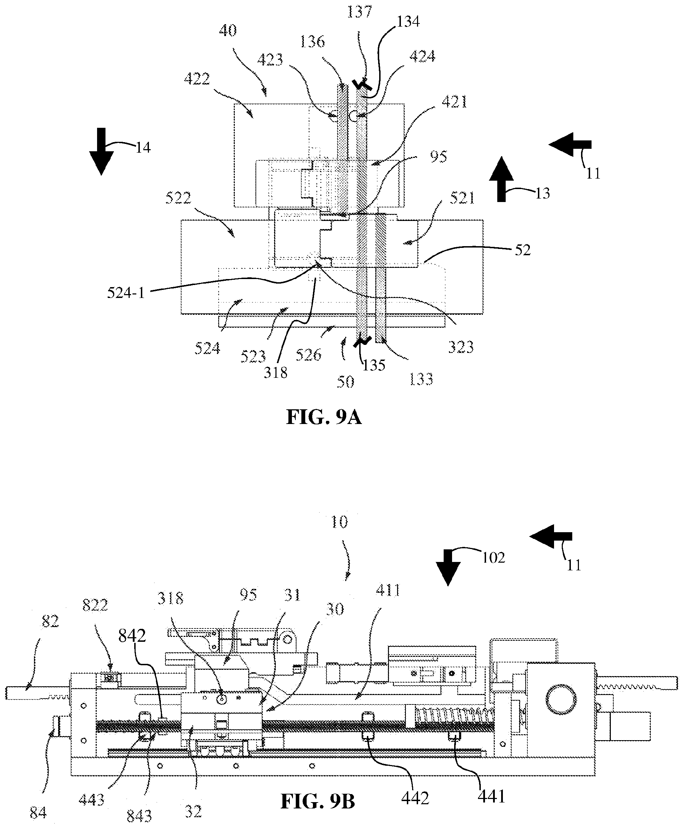

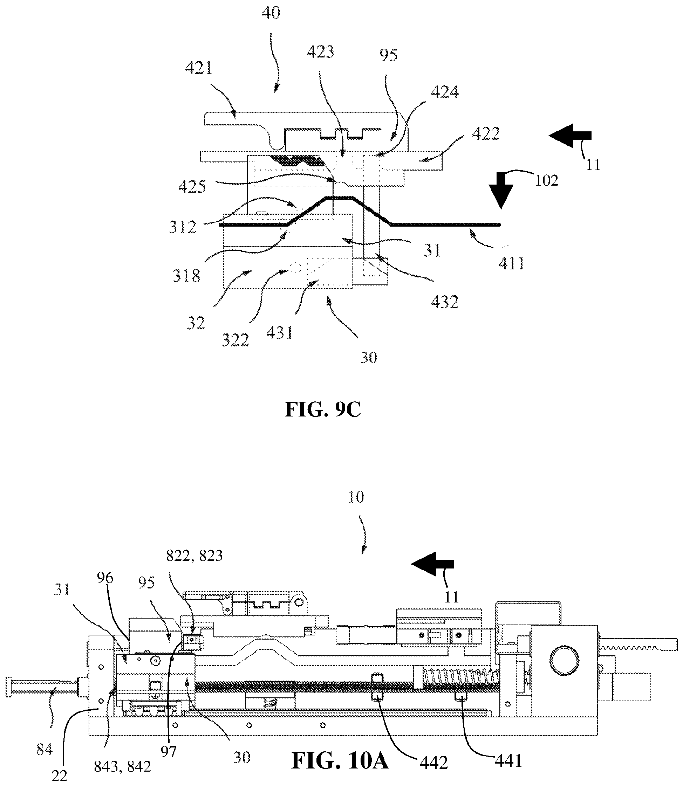

The right tube clamp 521 is provided on top of the welding mechanism assembly 52. The welding mechanism assembly 52 is laterally displaceable in leftward 13 and rightward 14 directions (i.e., perpendicular to the forward 11 and backward 12 directions), as indicated in FIG. 1A. The right tube clamp 521 is moved laterally leftward 13 towards the left tube clamp 421 after the retained lengths 134, 135 have been axially aligned and the wafer 95 has been moved away from contact with the retained lengths 134, 135. Rightward 14 lateral movement returns the right tube clamp 521 to the start position. By moving the right tube clamp 521 leftward 13 towards the left tube clamp 421, as shown in FIG. 9C, the melted cut surfaces of the axially aligned retained lengths 134, 135 are brought into contact with each other and held in place for a predetermined amount of time in order for welding of the two retained lengths 134, 135 to occur in order to form a single, joined tubing 137. The welding mechanism assembly 52 may further comprises a welding mechanism clamp mounting plate 522 on which the right tube clamp 521 is fixedly mounted, and a welding mechanism side plate 523 for fixedly connecting the welding mechanism clamp mounting plate 522 with the right cam plate 51. Further description on additional features of the welding mechanism clamp mounting plate 522 will be given below.

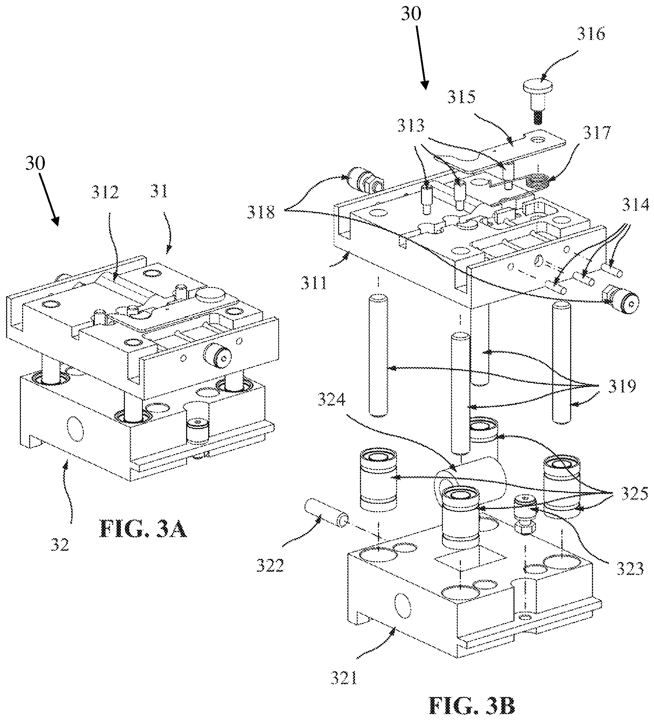

In an exemplary embodiment, the carriage 30 comprises an upper carriage assembly 31 mounted on top of a lower carriage assembly 32. Upper carriage guide shafts 319 affixed to the upper carriage assembly 31 are aligned with and slidably inserted in corresponding lower carriage bearing bushings 325 affixed in the lower carriage assembly 32 (FIGS. 3A and 3B), thereby allowing the upper carriage assembly 31 to move upward 101 and downward 102 relative to the lower carriage assembly 32 as the guide shafts 319 move within the bushings 325. In alternative embodiments, the guide shafts may be affixed to the lower carriage assembly 32 while bearing bushings that engage the guide shafts are affixed in the upper carriage assembly 31. The upper carriage assembly 31 is thus in vertically oriented sliding engagement with the lower carriage assembly 32, wherein both the upper and lower carriage assemblies 31, 32 are together moveable forward 11 and backward 12 and only the upper carriage assembly 31 is moveable upward 101 and downward 102.

In an exemplary embodiment, the lead screw 26 and motor 27 that move the carriage 30 forward 11 and backward 12 are part of a base mechanism assembly 20 that further comprise a linear guide carriage 24 on which the lower carriage assembly 32 is affixed, and linear guide rails 25 along which the linear guide carriage 24 is slidably engaged. The linear guide rails 25 are fixedly attached to a base plate 21 of the device 10. The lead screw 26 is rotatably supported parallel with the base plate 21 at its two ends by an upstanding front plate 22 and an upstanding back plate 23 that are fixedly mounted at front and back ends of the base plate 21 respectively. The carriage 30 travels between the front plate 22 and the back plate 23. A lead screw nut 324 of the lead screw 26 is provided within a lower carriage block 321 of the lower carriage assembly 32 (FIG. 3B) and prevented from rotating by the lower carriage block 321, so that the lower carriage assembly 32 is moved linearly forward 11 and backward 12 when the lead screw 26 is rotated by rotation of the motor 27.

The upper carriage assembly 31 is provided with first and second upper carriage cam followers 318 on left and right sides of the upper carriage assembly 31 (FIGS. 3 A and 3B). The upper carriage cam followers 318 are rotatable relative to the upper carriage assembly 31 but not otherwise movable relative to the upper carriage assembly 31. In an exemplary embodiment, the upper carriage cam followers 318 comprise rollers having horizontal and lateral axes of rotation, i.e., the axes of rotation are parallel with the left 13 and right 14 directions described above, i.e., perpendicular to the longitudinal forward 11 and backward 12 directions. The upstanding left and right cam plates 41, 51 are provided on left and right sides respectively of the carriage 30. The left and right cam plates 41, 51 are each provided with a cam track 411, 511 respectively on each of their inner surfaces that face the carriage 30 moving between them. The cam tracks 411, 511 preferably mirror each other. The upper carriage cam followers 318 rotatably engage upward 101 facing surfaces of the upper cam tracks 411, 511 to guide movement of the upper carriage assembly 31 according to the profile of the upper cam tracks 411, 511. The upper carriage assembly 31 is thus also supported by the upper carriage cam followers 318 resting on the cam tracks 411, 511 of the left and right cam plates 41, 51.

The profile of the cam tracks 411, 511 can be seen in greater detail in FIGS. 5A, 6B, 7B, 8B and 9B. Each cam track 411, 511 is preferably a continuous groove made in the left and right cam plates 41, 51 respectively in which the upper carriage cam followers 18 are rotatably engaged. As indicated in FIG. 5A, each cam track 411, 511 comprises: a first horizontal vector HI for horizontal movement of the upper carriage assembly 31; a first angled ramp AR1 that is angled upward 101 in the forward 11 direction until it reaches a pre-determined height, the first angled ramp AR1 provided forward 11 of the first horizontal vector HI; a second horizontal vector H2 that continues for a pre-determined horizontal length at the pre-determined height, the second horizontal vector H2 provided forward 11 of the first angled ramp; a second angled ramp AR2 that is angled downward 102 in the forward 11 direction until it reaches a level in line with the first horizontal vector HI, the second angled ramp AR2 provided forward 11 of the second horizontal vector H2; and a third horizontal vector H3 that is a horizontal continuation at the height of the first horizontal vector HI, the third horizontal vector H3 provided forward 11 of the second angled ramp AR2.

The cam tracks 411, 511 pass through four sections of the device 10, namely: a loading area, a heating area, a cutting area and an extraction area. The loading area is where the wafer 95 is mechanically loaded onto the upper carriage assembly 31. The heating area is where the heating elements 611 (preferably provided in a heater mount assembly 60 that is fixedly connected to the base plate 21) come into contact with the wafer 95 for thermal transfer to heat the wafer 95 to the pre-determined working temperature. The cutting area is where the wafer 95 cuts the pre-positioned thermoplastic tubings 131, 132 between where the tubings 131, 132 are clamped in place by the left tube clamp 421 and right tube clamp 521. The cutting area is also where the first and second tube clamps 421, 521 realign and weld the retained ends 134, 135 of the cut tubings 131, 132. The extraction area is where the wafer 95 is mechanically removed from the device 10 after cutting.

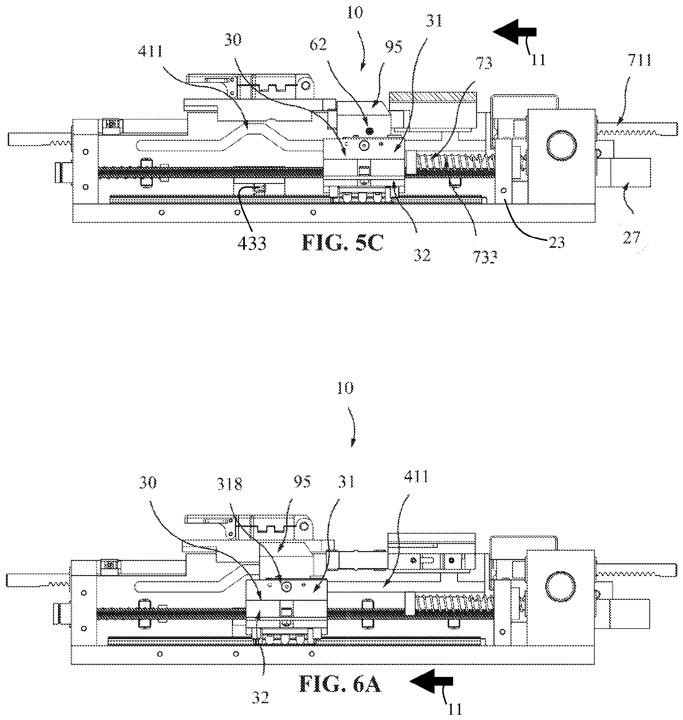

In an exemplary embodiment, in the start position before use of the device 10, the carriage 30 is at the heating area in a heating position aligned with a heating position sensor 442 provided on the device 10, as shown in FIG. IB. (FIG. 5C similarly shows the carriage 30 at the heating position but after having a wafer 95 loaded thereon.) Upon activation of the device 10, for example by depressing a start button 15, the motor 27 is activated and rotates in a first rotational direction which accordingly rotates the lead screw 26 in the first rotational direction. Rotation of the lead screw in the first rotational direction linearly translates the lead screw nut 324 in the lower carriage assembly 32 along the lead screw 26 in the backward direction 12, pulling the carriage 30 in the backward 12 longitudinal direction. The carriage 30 is thus driven in the backward longitudinal direction 12 from the heating position sensor 442 to a loading position aligned with a loading position sensor 441 provided on the device 10 (FIG 5B). The motor 27 is programmed to stop rotating, thereby stopping the carriage 30 momentarily at the loading position, when a lower carriage locating pin 322 provided on the lower carriage assembly 32 interfaces with the loading position sensor 441. In an exemplary embodiment, the locating pin 322 may comprise a light blocker pin while various position sensors such as the heating position sensor 442 and the loading position sensor 441 may comprise photosensors.

As the carriage 30 reverses in the backward 12 direction into the loading area, this activates or triggers the loading mechanism 70 to load a wafer 95 from the wafer cartridge 90 onto the upper carriage assembly 31. The loading mechanism 70 may comprise a secondary cam track, a rack and pinion mechanism, or any other appropriate mechanism that converts backward 12 linear motion of the carriage 30 to forward 11 (or other direction) motion of the loading mechanism 70 that loads the wafer 95 from the wafer cartridge 90 onto the upper carriage assembly 31.

In an exemplary embodiment as can be seen in FIGS. IB, 5A and 5C, the loading mechanism 70 comprises a loading mechanism housing 71 fixedly connected to the base plate 21 and supporting a loading pinion gear 72, a loading upper rack assembly 711, and a loading lower rack assembly 73. The loading upper rack assembly 711 comprises a loading upper rack 712 that engages the loading pinion gear 72 and is supported by the loading mechanism housing 71. The loading upper rack assembly 711 also includes a loading block 713 fixedly attached to the loading upper rack 712 (FIG. 2B). The loading lower rack assembly 73 comprises a loading lower rack 731 that is supported by the loading mechanism housing 71 and that also engages the loading pinion gear 72. The loading lower rack assembly 73 includes a loading lower rack plunger head 732 provided at a forward 11 end of the loading lower rack 731 and facing the carriage 30, and a loading lower rack spring 733 provided about the loading lower rack 731 between the loading lower rack plunger head 732 and the back plate 23. In this embodiment, the loading mechanism 70 is thus configured as a rack and pinion mechanism, where the loading lower rack 731 is biased towards a forward position towards the carriage 30 by the loading lower rack spring 733 when the carriage 30 is at the start position, i.e., the heating position.

When the device 10 is activated and a cutting cycle is started, the loading lower rack 731 is pushed backward 12 into the loading mechanism housing 71 from the forward position to a backward position by the carriage 30 when the carriage 30 is in contact with lower rack plunger head 732 and the carriage 30 moves in the backward longitudinal direction 12 from the heating position into the loading position (FIG. 5A). Accordingly, the loading pinion gear 72 is driven into rotational motion by the lower rack 731 moving backward 12. This rotational motion of the pinion gear 72 in turn drives and extends the loading upper rack 712 and attached loading block 713 in the opposite direction, i.e., forward 11. As the loading block 713 continues moving forward 11, the loading block 713 comes into contacts with a rear edge 97 of a frontmost wafer 95 in the wafer cartridge 90, pushes and slides the wafer 95 forward 11 out of the wafer cartridge 90, and loads the wafer 95 onto the upper carriage assembly 31, as illustrated in FIG. 5B.

As the wafer 95 is loaded onto the upper carriage assembly 31 via the loading block 713, a lower edge of the wafer 95 is slid into a wafer support slot 301 provided on an upper carriage block 311 of the upper carriage assembly 31, between wafer guide pins 313 on the upper carriage block 311 that guide movement and positioning of the wafer 95 in the wafer support slot 301, as can be seen in FIG. 3A and 3B. A wafer latch 315 rotatable about a wafer latch pivot screw 316 having a vertical axis of rotation is provided on the upper carriage block 311. The wafer latch 315 has a tip extending into the wafer support slot 301. As the wafer 95 slides into the wafer support slot 301, a front edge 96 and side of the wafer 95 come into contact with the tip of the wafer latch 315 to displace and rotate the wafer latch 315 about the wafer latch pivot screw 316. As the tip of the wafer latch 315 is biased towards the wafer support slot 301 by a wafer latch spring 317 provided in the upper carriage block 31 (FIG. 3B), the tip of the wafer latch 315 remains in contact against the side of the wafer 95 as the wafer slides along the wafer support slot 301 until the tip of the wafer latch 315 penetrates and engages a wafer latch slot 956 provided in the wafer 95, at which point the wafer 95 is considered properly loaded onto the carriage 30 and backward movement of the wafer 95 is prevented by the wafer latch 315. The wafer latch slot 956 is located adjacent the bottom edge of the wafer 95.

After the wafer 95 has been loaded onto the carriage 30, the carriage 30 is then driven in the forward longitudinal direction 11 with the wafer 95 into the heating area by the lead screw 26 upon rotation of the motor 27 in a second rotational direction and stops at the heating position when the lower carriage locating pin 322 interfaces with the heating position sensor 442. This forward 11 motion of the carriage 30 also resets the loading mechanism 70 to prepare a next wafer 95 to be loaded from the wafer cartridge 90 as the loading lower rack spring 733 returns the loading lower rack 731 to the forward position.

At the heating position, the wafer 95 is positioned between a pair of heater assemblies 61, as shown in FIG 5C. The pair of heater assemblies 61 at the heating area each comprises heating elements 611, an insulating strip 612 and a heater cantilever spring 614 (FIG. 2A). The heater assemblies 61 are positioned in the path of the carriage 30, on either side of the wafer 95 on the upper carriage assembly 31, at a location where the heating position sensor 442 is provided. The heater assemblies 61 are supported by a heater mount assembly 60 that is positioned across the path of the carriage 30. The heater cantilever spring 614 compresses laterally inwards towards the wafer 95 to allow the heating elements 611 to completely press onto a wafer heating area 954 of the wafer 95, while the insulating strip 612 between the heating element 611 and the heater cantilever spring 614 reduces the amount of possible thermal energy lost through conduction. When the lower carriage locating pin 322 interfaces with the heating position sensor 442, the motor 27 is programmed to stop rotating so that the wafer 95 remains stationary between the heater assemblies 61 in contact with the heating elements 611. At this position, the electrical control circuit board 17 activates the heating elements 611 to heat up the wafer 95.

A wafer temperature sensor 62 placed near the wafer 95 reads the temperature of the wafer 95 as it is being heated up. In an exemplary embodiment, the temperature sensor 62 may be provided on the upper carriage block 311 (FIGS. 3B and 3C), for example on one of the wafer guide pins 313. When the wafer 95 is heated to a sufficient working temperature as determined by the wafer temperature sensor 62, the motor 27 is activated by the electrical control circuit board 17 to drive the carriage 30 and wafer 95 in the forward longitudinal direction 11 from the heating position sensor 442 to the cutting position, to begin the cutting and welding process. The motion at this stage of the operation is shown in FIGS. 6A, 6B and 6C.

In an exemplary embodiment as shown in FIGS. 4A and 4B, the wafer 95 comprises a folded metal sheet and may be made of a metallic material such as copper. The wafer 95 may thus be formed by, not exclusively, folding a metal sheet with dimensionally disproportionate sections. Thus, the fold of the metal sheet is at the bottom edge 958 of the wafer 95 (FIG. 4A). The wafer 95 comprises three regions, namely: a wafer working area 953, a wafer heating area 954 and a wafer holding area 955 (FIG. 4B). This configuration of the wafer 95 allows the working area 953 to be a single layer while the heater contact heating area 954 and holding area 955 comprise two or more layers of the folded metal sheet. The working area 953 is required to be thin to increase precision and reduce loss of material when cutting and welding the thermoplastic tubing. A rear comer of the working area 953 is profiled so as to allow uniform contact with the melted cut ends of the retained lengths of tubings 134, 135 as the wafer 95 leaves the tubing 131, 132 in a way that allows minimal tubing residue on the wafer 95 to contaminate the cut tubing 134, 135. This profile, not exclusively this particular design, varies depending on the profile of the corresponding cam tracks 411, 511 used in the device 10.

As mentioned above, the profile of the metal sheet is such that when folded, a cutting portion of the wafer 95 remains as a single layer section 951 while the remainder of the wafer 95 is a multiple layer section 952 comprising two layers of the metal sheet connected by a fold. In an exemplary embodiment as shown in FIG. 4A, the fold is along the front edge 96 of the wafer 95. In alternative embodiments, the fold may be along the bottom edge 958 or rear edge 97 of the wafer 95. The working area 953 is where both lengths of thermoplastic tubing 131, 132 come into contact with the wafer 95 during the cutting and welding process. Thus, the wafer working area 953 is the single layer section 951 that comprises the single layer of material that is required to cut the thermoplastic tubing 131, 132. The heating area 954 is where the heating elements 611 of the heater assembly 61 press onto the wafer 95 and heat it to the preset temperature. The holding area 955 is where the wafer 95 is held in the wafer support slot 301 on the upper carriage assembly 31 by the wafer guide pins 313 and wafer support pins 314 (FIG. 3B) that may be provided under the wafer support slot 301 in the upper carriage block 311. The holding area 955 further comprises the wafer latch slot 956 to engage the wafer latch 315 and secure the wafer 95 on the upper carriage assembly 31, as described above. Additional features to allow or enhance automatic loading and unloading mechanisms may be included at the holding area 955. The holding area 955 is preferably also multi-layered, giving the wafer 95 increased structural strength to be carried throughout the entire process and prevents the wafer 95 from bending as thin metal sheets may soften when heated to high temperatures.

With sufficient space at the holding area 955, other features such as one-time-use markers 957 can be incorporated to prevent reuse of the wafers. Such markers 957 can be read by sensors built into the device 10 to reject wafers that have been used before. Markers 957 may comprise, and are not limited to, RFID tagging, fluorescence coating that is either altered by high temperature or by mechanical means. In addition, electrical control circuit board 17 may further be appropriately configured to detect, via sensors (not shown), if the wafer 95 is new before proceeding to cut and weld the tubing 131, 132. This prevents contamination of materials from a previous tube connection to the tubing 131, 132 that are going to be connected.

In the wafer 95, the multilayered wafer heating area 954 (FIG. 4A) has a larger thermal mass than the wafer working area 953 that comprises the single layer section 951. The wafer heating area 954 acts as a thermal capacitive feature to decrease rate of heat loss at the wafer working area 953 when the wafer 95 leaves the heater assemblies 61 for the cutting and welding process. As further heat loss will occur during the cutting and welding process, the multilayered wafer heating area 954 and wafer holding area 955 of the wafer 95 will continue to supply thermal energy or heat to the wafer working area 953 as the stored thermal energy shifts from a higher potential region 54, 955 to a lower potential region 953.

Notably, some parts of the carriage 30 (e.g., those that come into contact with the wafer 95 or are close to the heating elements 611 when the carriage 30 is at the heating position) may be made of a heat resistant polyimide-based plastic such as Vespel^{®} (e.g. the wafer guide pins 313) or zirconia (e.g. the wafer support pins 314) so that they have high thermophysical stability and thus present minimal dimensional changes when exposed to heat from the heating elements 611 while minimizing heat loss from the wafer 95 through contact with the wafer 95.

As described above, while the wafer 95 is between the heating elements 911, a temperature sensor 62 checks the wafer 95 to ensure it is heated to the required temperature. After heating, the wafer 95 is then driven forward 11 to the cutting area by rotating the motor 27 in the second rotational direction to move the carriage 30 forward 11. As the carriage 30 moves forward 11, the upper carriage cam followers 318 move along the first horizontal vector HI of the cam tracks 411, 511. When the upper carriage cam followers 318 are at the level of the first horizontal vector HI, the cutting upper edge 959 of the wafer 95 is lower than both the tubings 131, 132 that are clamped on the top of the device 10 by the left and right tube clamps 421, 521 (FIGS. 6A and 6B).

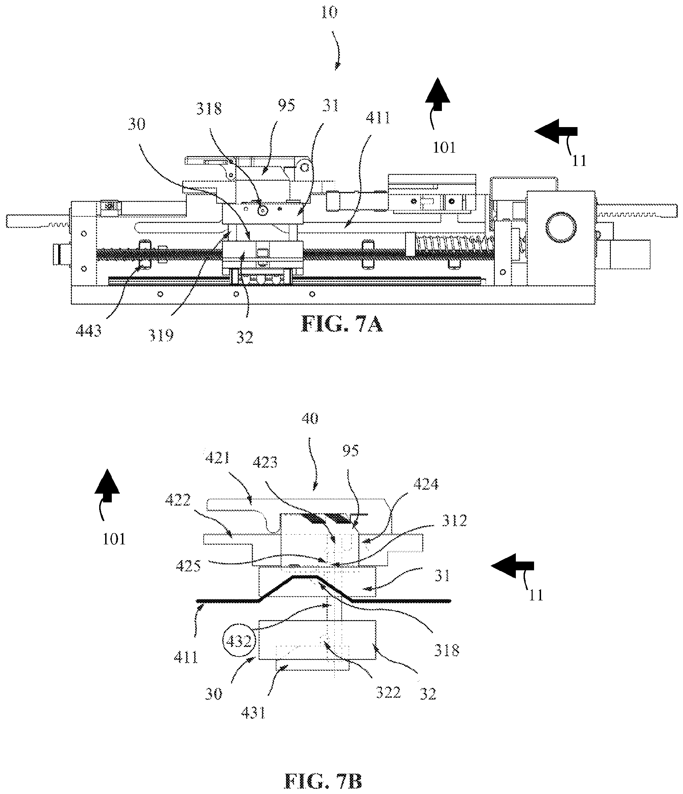

As the carriage 30 continues to move forward 11, the upper carriage cam followers 318 reach and engage the first angled ramp AR1 of the cam tracks 411, 511 to raise the upper carriage assembly 31 up along the angled ramp AR1 of the cam tracks 411, 511 as the upper carriage assembly 31 is pulled forward by the lower carriage assembly 32. This allows the upper edge 959 of the wafer 95 to maintain a parallel alignment with both lengths of the thermoplastic tubing 131, 132 in order to cut them both at the same time from below up as the wafer 95 is simultaneously moved forward 11 and raised, without any rotational movement of the upper or lower carriage assemblies 31, 32. The upper carriage guide shafts 319 are positioned near the comers of the upper carriage block 311 of the upper carriage assembly 31, allowing the upper carriage assembly 31 to maintain a parallel alignment to the tubing 131, 132 as the upper carriage assembly 31 ascends in an angled vector corresponding to that of the first angled ramp AR1. This motion at this stage of the operation is shown in FIGS. 7A, 7B and 7C.

As the upper carriage assembly 31 travels along the first angled ramp grooves AR1 of the cam tracks 411, 511, the upper carriage assembly 31 is simultaneously moving both forward 11 and upward 101 while the lower carriage assembly 32 only moves forward. During this time, a pin provided on the lower carriage assembly 32 (which may be the same pin as the lower carriage locating pin 322) engages a displacement lock mechanism 43 provided on the displacement mechanism assembly 42 (FIG. 7B). The displacement lock mechanism 43 is configured to lock and unlock the displacement mechanism assembly to prevent and allow movement of the displacement mechanism assembly 42 respectively. When the displacement mechanism assembly 42 is locked by the displacement lock mechanism 43, such as in the start position, the displacement mechanism assembly 42 cannot linearly translate in the forward 11 or backward 12 directions.

In an exemplary embodiment, the displacement lock mechanism 43 comprises a vertically moveable and longitudinally immoveable displacement lock cam guide 431, a displacement lock pin 432 having a lower end fixedly attached to the displacement lock cam guide 431, a displacement lock spring 433 (visible in FIGS. 5A and 5C) that biases the displacement lock cam guide 431 and pin 432 upward 101, and a front pin slot 423 and a back pin slot 424 both provided on the displacement mechanism assembly 42. The front pin slot 423 and back pin slot 424 each separately engage the upper end of the displacement lock pin 432 at different times to obstruct movement of the displacement mechanism assembly 42. The front and back pin slots 423, 424 may be provided on an underside of the displacement mechanism clamp mounting plate 422 of the displacement mechanism assembly 42, the front pin slot 423 being located forward 11 of the back pin slot 424 in the longitudinal direction.

Notably, while the upper carriage cam followers 318 engage the first horizontal vector HI of the cam tracks 411, 511, a free upper end of the displacement lock pin 432 is located in and engages the front pin slot 423, thereby locking the displacement mechanism assembly 42 and preventing it from being displaced forward 11 or backward 12.

In an exemplary embodiment, an upward 101 facing surface of the lock mechanism cam guide 431 that interfaces and engages with the lower carriage locating pin 322 has a similar profile to that of the cam track 411,511, allowing the displacement lock mechanism 43 to be activated relative to the position of the carriage 30. As the carriage 30 passes the displacement mechanism assembly 42 while moving forward 11, the lower carriage locating pin 322 on the lower carriage assembly 32 pushes down the displacement lock cam guide 431, thereby lowering the displacement lock pin 432 and disengaging the displacement lock pin 432 from the front pin slot 423 of the displacement mechanism clamp mounting plate 422 and unlocking the displacement mechanism assembly 42 to allow forward 11 or backward 12 movement. The lower carriage locating pin 322 thus may comprise a roller similar to the upper carriage cam followers 318, that has a horizontal and lateral axis of rotation, in order to rotatably engage the upward 101 facing surface of the lock mechanism cam guide 431.

In an exemplary embodiment, the displacement lock pin 432 is simultaneously lowered from the front pin slot 423 as the upper carriage assembly 31 is raised to the predetermined height (FIGS. 7A to 7C). This means that the lower locating pin 322 is engaging and pushing down the displacement lock cam guide 431 and bringing down the displacement lock pin 432 with it at the same time that the upper carriage cam followers 318 are going up the first angled ramps AR1 of the cam tracks 411, 511. During this time, as the upper carriage assembly 31 moves both forward 11 and upward 101 while the displacement lock pin 432 is being lowered, the wafer 95 is also moved both forward 11 and upward 101 to cut both tubings 131 and 132 at the same time from below up, due to the upper edge 959 of the heated wafer working area 953 of the wafer 95 maintaining a parallel alignment relative to the arrangement of both tubings 131, 132 as can be seen in both FIGS. 6B and 7B.

Notably, during cutting, while the displacement lock pin 432 is being lowered out of the front pin slot 423 as the wafer 95 is being raised to cut through both the two tubings 131, 132, the free upper end of the displacement lock pin 432 is still has not fully out of the front pin slot 423. Thus, the displacement mechanism assembly 42 is still locked and prevented from moving longitudinally, thereby keeping the two tubings 131, 132 stationary while the working area 953 of the wafer 95 passes through them for a clean and controlled cut. After cutting, the wafer 95 remains between and in contact with both the retained length 134 of the first tubing 131 and the retained length 135 of the second tubing 132, thereby maintaining a sterile closed loop for both retained lengths 134, 135. After cutting of the two tubings 131, 132, the retained lengths 134, 135 remain clamped by the left tube clamp 421 and right tube clamp 521 respectively, while waste ends 133, 136 of the first and second tubings 131, 132 remain clamped by the right tube clamp 521 and the left tube clamp 421 respectively (FIG. 7C).

As the carriage 30 continues to move forward 11, when the upper carriage cam followers 318 reach the start of the second horizontal vector H2 in the cam tracks 411, 511, the displacement lock pin 432 becomes fully disengaged from the front pin slot 423 to allow longitudinal movement of the displacement mechanism assembly 42. At this point, both tubings 131, 132 have been cut, and a displacement catch 312 provided on the upper carriage assembly 31 engages a displacement notch 425 that is provided on the displacement mechanism clamp mounting plate 422 of the displacement mechanism assembly 42. The displacement catch 312 and displacement notch 425 are located such that they engage each other only when the displacement lock pin 432 has been fully disengaged from the front pin slot 423 to allow longitudinal movement of the displacement mechanism assembly 42. In this way, the carriage 30 is automatically and mechanically connected to the left tube clamp 421 to move the left tube clamp 421 forward when the carriage 30 moves forward after the first and second tubings 131, 132 have been cut.

In an exemplary embodiment, the displacement catch 312 comprises a ridge protruding from a top face of the upper carriage block 311 along a lateral axis, while the displacement notch 425 comprises a female interface or depression on an underside of the displacement mechanism clamp mounting plate 422 to receive and engage the displacement catch 312 therein (FIG. 7B). In this way, the upper carriage assembly 31 can contact and transmit a forward 11 longitudinal force on the displacement mechanism assembly 42 as the carriage 30 continues to be moved forward 11 with the upper carriage cam followers 318 engaging the second horizontal vector H2 of the cam tracks 411, 511, thereby pulling the displacement mechanism assembly 42 with it forward 11 (along the longitudinal axis), after the displacement lock pin 432 has been lowered and disengaged from the front pin slot 423. In alternative embodiments, the displacement catch 312 may be provided on the underside of the displacement mechanism clamp mounting plate 422 while the displacement notch 425 is provided on the top face of the upper carriage block 311, for example.

As the displacement mechanism assembly 42 is pulled forward 11 by the upper carriage assembly 31 moving forward 11, this aligns the retained lengths 134, 135 of the thermoplastic tubing to be joined (FIGS. 8 A to 8C), as already described above, while the wafer 95 remains in contact with the cut melted ends of the retained lengths 134, 135 of the first and second tubings 131, 132. When the retained length 134 of the first tubing 131 that is clamped by the left tube clamp 421 has been displaced forward 11 to be axially aligned with the retained length 135 of the second tubing 132 that is clamped by the right tube clamp 521, the upper carriage assembly 31 follows the second angled ramps AR2 in the cam tracks 411, 511 downward 102 and forward 11 to move the wafer 95 away from the retained lengths 134, 135 of the cut tubings 131, 132.

As the upper carriage assembly 31 follows the second angled ramps AR2 to move forward 11 and downward 102, at the same time, a lower carriage cam follower 323 fixedly provided on the lower carriage assembly 32 travels over a step 524-1 of a welding cam guide 524 provided on the right cam plate 51 as shown in FIG. 9A. The step 524-1 is shown in perspective in FIG. 2B and extends laterally outward 14 away from the carriage 30. In an exemplary embodiment, the lower carriage cam follower 323 comprises a roller having a vertical axis of rotation that is fixedly provided on a right side of the lower carriage assembly 32 such that relative motion of the lower carriage cam follower 323 with the lower carriage assembly 32 comprises only rotation of the lower carriage cam follower 323. The lower carriage cam follower rotatably engages the welding cam guide 524. The welding cam guide 524 faces the path of travel of the carriage 30, and may comprise a laterally projecting profiled surface on the inner surface of the right cam plate 51 along which the lower carriage cam follower 323 rotates. The welding cam guide 524 may be provided on the right cam plate 51 below the cam track 511, as can be seen in FIG. 2B. The right cam plate 51 is biased laterally towards the left cam plate 41 by one or more welding mechanism springs (not shown) that are provided between the right cam plate 51 and a welding mechanism back plate 526 that is fixedly connected to the base plate, i.e., the welding mechanism back plate 526 is immovable. In this way, the welding cam guide 524, right cam plate 51, welding mechanism mounting plate 522, and right tube clamp 521 that are all fixedly connected to each other are all laterally displaceable leftward 13 and rightward 14 from the welding mechanism back plate 526 that is fixedly connected to the base plate 21 of the device 10. To facilitate this, the welding mechanism mounting plate 522 may be connected by linear guide rails 528 (that allow lateral movement 13, 14 of the welding mechanism mounting plate 522) to an immoveable upstanding wall 50 (FIG. 1A) of the device 10.

Notably, the longitudinal travel path of the lower carriage cam follower 323 is configured to interfere with the laterally projecting surface of the welding cam guide 524. Therefore, it can be said that lateral displacement and positioning of the right tube clamp 521 (that is fixedly connected to the right cam plate 51) are determined by the location of the lower carriage cam follower 323 along the profiled surface of the welding cam guide 24.

As can be seen in FIG. 2B, the step 524-1 on the welding cam guide 524 is provided on the laterally surface of the welding cam guide 524. The carriage 30 (including the lower carriage cam follower 323) is unable to move laterally but the welding cam guide 524 (that is in relative rolling contact against the lower carriage cam follower 323) is laterally displaceable rightward 14 by the lower carriage cam follower 323, against the leftward 13 bias provided by the one or more welding mechanism springs. Thus, when the lower carriage cam follower 323 moves forward 11 to engage the step 524-1 on the welding cam guide 524, the compressed welding mechanism springs 525 displace the right cam plate 51 (and accordingly the right tube clamp 521) in a lateral direction 13 towards the wafer 95. In this way, the device 10 automatically and mechanically displaces the right tube clamp 521 towards the left tube clamp 421 (i.e., leftward 13) when the carriage 30 moves forward 11 after the retained length 134 of the first tubing 131 has been axially aligned with a retained length 135 of the second tubing 132. This leftward 13 lateral movement of the right tube clamp 521 towards the left tube clamp 421 moves the melted cut ends of the retained length 134 of the first tubing 131 and the retained length 135 of the second tubing 132 into contact with the surface of the wafer working area 953 to compensate for tubing material lost during the cutting process and to maintain the sterile closed loop with the retained lengths 134, 135 of the cut tubing 131, 132.

As the carriage 30 continues in the forward longitudinal direction 11, the upper carriage assembly 31 follows the cam track 411, 511 down the second angled ramp AR2, thereby lowering the wafer 95 from between the tubings 131, 132 so that the wafer 95 is no longer in contact with the tubings 131, 132. This motion also causes the displacement catch 312 of the upper carriage assembly 31 to disengage from the displacement notch 425 on the displacement mechanism clamp mounting plate 422 (FIG. 9C). At the same time, the lower carriage locating pin 322 on the lower carriage assembly 32 has moved past and is disengaged from the displacement lock cam guide 431, thereby causing the upper free end of the displacement lock pin 432 to be raised into the back pin slot 424 of the displacement mechanism clamp mounting plate 422 (under the upward 101 biasing action of the displacement lock spring 433) to hold the displacement mechanism assembly 42 in place (FIG. 9C). Concurrently, the one or more welding mechanism springs continue pushing the right cam plate 52 (and accordingly the right tube clamp 521) laterally leftward 13 towards the left tube clamp 421, moving the melted cut end of the retained length 135 of the second tubing 132 leftward 13 against the axially aligned melted cut end of the retained length 134 of the first tubing 131 to form a single tubing 137 (as indicated in FIG. 9A). The left tube clamp 421 and right tube clamp 521 are left to remain in this position to keep the melted cut ends of both retained lengths of tubings 134, 135 pressed together to allow the welded tubing 137 to cool and fuse while the carriage 30 continues moving forward 11 along the third horizontal vector H3.

Thus, it can be said that as the wafer 95 is moved forward 11 and downward 102 away from the cut tubings 131, 132 by the continued forward 11 movement of the carriage 30 that also lowers the upper carriage assembly 31, the welding mechanism assembly 52 is triggered by the lower carriage assembly 32 to move the right tube clamp 521 laterally 13 towards the left tube clamp 421. This moves the retained length 135 of the second tubing 132 (that is clamped by the right tube clamp 521) inwards laterally 13 towards the forwardly 11 displaced retained length 134 of the first tubing 131 (that is clamped by left tube clamp 421) for welding.

The device 10 may be further provided with an extraction mechanism 80 to automatically remove the used wafer 95 from the device 10 after the tubing 131, 132 have been cut. Preferably, further forward 11 movement of the carriage 30 along the third horizontal vector H3 moves the carriage 30 to the extraction area, where alignment of the lower carriage locating pin 322 with an extraction position sensor 443 provided on the device 10 halts further forward 11 movement of the carriage 30. When the carriage 30 is driven horizontally forward 11 into the extraction area, the extraction mechanism 80 (may be similar to that of the loading mechanism 70) is triggered and engages the wafer 95. When the device 10 is reset (for example by pressing a reset button 16), the carriage 30 returns to the starting position which causes the extraction mechanism 80 to remove the wafer 95 from the carriage 30.

In an exemplary embodiment, the extraction mechanism 80 comprises an extraction mechanism housing 81 fixedly connected to the base plate 21 and supporting an extraction pinion gear 83, an extraction upper rack assembly 82, and an extraction lower rack assembly 84. The extraction upper rack assembly 82 comprises an extraction upper rack 821 that engages the extraction pinion gear 83 and is supported by the extraction mechanism housing 81. The extraction upper rack assembly 82 also includes an extraction linkage 822 that is fixedly attached to the extraction upper rack 821. An extraction catch 823 is moveably connected to the extraction linkage 822 via an extraction catch spring 824 provided about an extraction catch shaft 825 that is fixedly connected to the extraction linkage 822. The extraction catch 823 is laterally displaceable leftward 13 and rightward 14 relative to the extraction linkage 822 by orientating the longitudinal axes of the extraction catch spring 824 and catch shaft 825 in a horizontal and lateral direction.

In the start position, the extraction linkage 822 and extraction catch 823 are adjacent the e 22 of the device 10, as can be seen in FIG. IB. The extraction lower rack assembly 84 comprises an extraction lower rack 841 that drives the extraction pinion gear 83 to rotate when the extraction lower rack 841 translates linearly in the longitudinal direction. The extraction lower rack assembly 84 includes an extraction lower rack plunger head 842 provided at one end of the extraction lower rack 841 and facing the carriage 30, and an extraction lower rack spring 843 provided about the extraction lower rack 841 between the extraction lower rack plunger head 842 and the front plate 22. In this embodiment, the extraction mechanism 80 is thus configured as a rack and pinion mechanism, where the extraction lower rack 841 is biased towards a backward position towards the carriage 30 by the extraction lower rack spring 843 when the carriage 30 is at the start position, i.e., the heating position.

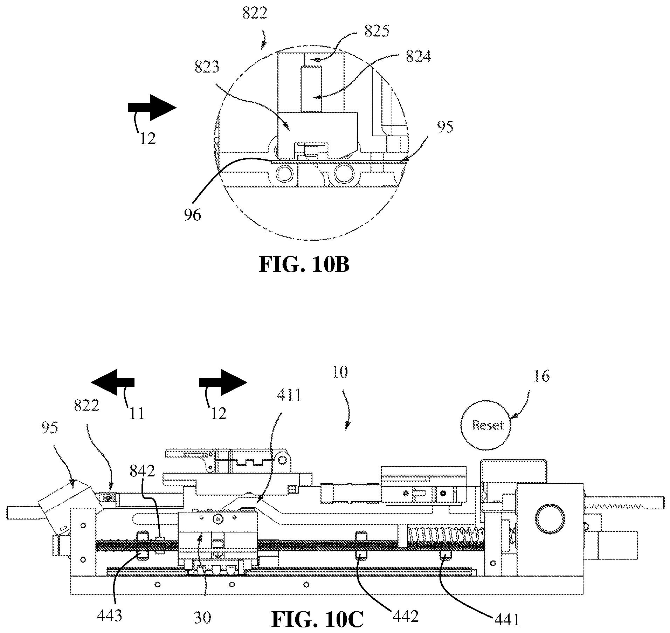

When the extraction mechanism lower rack 841 is pushed forward into the extraction mechanism housing 81 by the carriage 30 as the carriage 30 moves in the forward longitudinal direction 11 until the lower carriage locating pin 322 interfaces with the extraction position sensor 443 (FIG. 10A), the extraction mechanism pinion gear 83 is driven into motion, thereby extending the extraction upper rack 821 and attached extraction linkage 822 in the opposite direction, i.e., backward 12. As the extraction linkage 822 moves backward, 12, the extraction catch 823 comes into contact with a front edge 96 of the wafer 95 on the upper carriage 30 and is displaced leftward 13 towards the extraction linkage 822 along the extraction catch shaft 825, thereby compressing the extraction catch spring 824 while the extraction catch 823 continues to slide backward 12 along the side of the wafer 95 (FIG. 10B) until the extraction catch 823 falls behind the rear edge 97 of the wafer 95 (FIG. 10A). When the extraction catch 823 is behind the rear edge 97 of the wafer 95, the compressed extraction catch spring 824 extends the extraction mechanism catch 823 out behind the wafer 95 in a lateral direction 14 to engage the rear edge 97 of the wafer 95.

In an exemplary embodiment, upon activation of a reset button 16 of the device 10, the motor 27 is rotated in a second rotational direction which drives the carriage 30 in the backward 12 longitudinal direction from the location of extraction position sensor 443 (FIG. IOC) towards the location of the heating position sensor 442. At the same time, the compressed extraction lower rack spring 843 pushes the extraction lower rack plunger head 842 in the backward 12 direction as the carriage 30 is driven backward 12, thereby returning the extraction linkage 822 and extraction catch 823 in the forward direction 11 to the start position adjacent the front plate 22. AS the extraction catch returns to the start position, it pulls the wafer 95 in the forward longitudinal direction 11, thereby disengaging the wafer latch 315 from the wafer latch slot

956 to slide the wafer 95 out of the upper carriage assembly 31 and also out of the device 10 through an extraction slot 221 provided in the front plate 22 (as can be seen in FIG. 1 A).

As the activation of all supplementary mechanisms such as the displacement mechanism assembly 42 and the extraction mechanism 80 are dependent on position of the carriage 30 along the linear guide rails 25, these mechanisms are also reset to their starting configurations as the carriage 30 follows the cam tracks 411, 511 in the reverse or backward 12 direction.

For example, when the upper carriage assembly 32 is going up the second angled ramps AR2 in the backward direction 12, the lower locating pin 322 is engaging and pushing down the displacement lock cam guide 431 and bringing down the displacement lock pin 432 with it at the same time so that the displacement lock pin 432 is lowered out of the back pin slot 424 of the displacement mechanism clamp mounting plate 422.

As the carriage 30 continues to move backward 12, when the upper carriage cam followers 318 reach the second horizontal vector H2 in the cam tracks 411, 511, the displacement lock pin 432 becomes fully disengaged from the back pin slot 424 to allow longitudinal movement of the displacement mechanism assembly 42. At this point, the displacement catch 312 once again engages the displacement notch 425 and the upper carriage assembly 31 can contact and transmit a backward 12 longitudinal force on the displacement mechanism assembly 42. As the carriage 30 continues to be moved backward 12 with the upper carriage cam followers 318 engaging the second horizontal vector H2 of the cam tracks 411, 511, this pulls the displacement mechanism assembly 42 with it backward 12 (along the longitudinal axis) to its starting position and the carriage 30 finally comes to rest at the heating area when the lower carriage locating pin 322 interfaces with the heating position sensor 442. Similarly, the lower carriage cam follower 32 travels along the second step 524-2 the first step 524-1 and the rest of the of the welding mechanism cam guide 524 in the backward direction 12, thereby returning the right cam plate 51 and right tube clamp 521 to their start positions.

To effect and control the above movements, the device 10 is provided with electrical controls performed by the electrical control circuit board 17 that is programmed to control operation of the device 10 according to the following exemplary sequence that describes one complete cycle of control of the device 10:
- upon activation of the start button 15, rotate the motor 27 in a first rotational direction to drive the carriage 30 from the start position in the backward longitudinal direction 12 at one or more programmed speeds to load a wafer 95 onto the carriage 30;
- when the lower carriage locating pin 322 interfaces with loading position sensor 441, stop and rotate the motor 27 in the second rotational direction to drive the carriage 30 carrying the wafer 95 in the forward longitudinal direction 11 at one or more programmed speeds;
- when the lower carriage locating pin 322 interfaces with the heating position sensor 442, stop the motor 27 and activate heating elements 611 to heat the wafer 95 to the pre-determined temperature;
- when the wafer temperature sensor 62 detects that temperature of the wafer 95 has reached the pre-determined temperature, stop heating elements 611 from further heating and rotate the motor 27 in the second direction to drive the carriage 30 in the forward longitudinal direction 11 at one or more programmed speeds to cut and join both tubings 131, 132;
- when the lower carriage locating pin 322 interfaces with the extraction position sensor 443, stop the motor 27;
- upon activation of the reset button 16, rotate the motor 27 in the first direction to drive the carriage 30 in the backward longitudinal direction 12 at one or more programmed speeds to extract the used wafer 95 from the carriage 30 and return the displacement mechanism assembly 42 and welding mechanism assembly 52 to their start positions;
- when the lower carriage locating pin 322 interfaces with the heating position sensor 442, stop the motor 27 as the carriage 30 has returned to the start position.

In the above-described device 10, it can be appreciated that only one motor 27 is needed to drive all the moving parts of the device 10 in their various different directions in order for the two tubings 131, 132 to be cut and joined to form a single length of tubing 137 while maintaining sterile conditions of the tubing 137. Even the loading and extraction of the cutting wafer 95 can be automatically performed on the device 10 using the same one motor 27. By mechanically coupling the longitudinal movement of the carriage 30 with the required longitudinal displacement of one of the two tube clamps 421, 521 to align the retained lengths of the two tubings 134, 135 for welding after cutting, and also mechanically coupling the longitudinal movement of the carriage 30 with the required lateral displacement of one of the two tube clamps 421, 521 to weld the aligned retained lengths of the two tubings 134, 135 together, only one motor 27 is required in the present device 10 to effect all the necessary movements to cut and weld the two tubings 131, 132 to form a single length of tubing 137. In this way, the device 10 is rendered compact and space-saving compared to currently available sterile tube connecting devices, thereby making the device 10 portable as it can be easily taken to various different locations for on-site use. The device 10 is also easy to control as it does not have multiple motors each controlling different parts moving in different directions that need to be coordinated and synchronized. Instead, the present device 10 is readily programmed by setting only the directions of rotation and stoppage of the one motor 27 for various predetermined intervals of time in order for all the different stages of the entire process to be performed by the device 10, including wafer loading, tube cutting, tube re-aligning, tube welding and wafer extraction.

Whilst there has been described in the foregoing description exemplary embodiments of the present invention, it will be understood by those skilled in the technology concerned that many variations and combination in details of design, construction and/or operation may be made without departing from the present invention. For example, while it has been described above that the left tube clamp is longitudinally displaced forward after the two tubings have been cut and the right tube clamp is thereafter laterally displaced leftward towards the left tube clamp to join the retained ends of the two tubings to form a single tubing, it should be understood that in alternative embodiments, it may be the right tube clamp that is longitudinally displaced forward after the two tubings have been cut and the left tube clamp is thereafter laterally displaced rightward towards the right tube clamp to join the retained ends of the two cut tubings to form a single tubing. While the loading mechanism assembly in the above-described embodiment and as shown in the figures comprises double racks and pinion actuator, in other embodiments, the loading mechanism assembly may alternatively comprise a cam and guide mechanism to translate the linear motion of the carriage assembly to a motion in a different vector to load the wafer. While aluminium, steel, copper, Vespel^{®} and zirconia have been mentioned above as the material that may be used for many of the components of the device, other appropriate materials may be used to form the components.

## Claims

1. A sterile tube connecting device (10) for joining a first tubing (131) with a second tubing (131, 132), the device (10) comprising:
a first tube clamp (421) longitudinally moveable forward and backward;
a second tube clamp (421, 521) spaced apart from the first tube clamp (421) and laterally moveable leftward and rightward perpendicular to forward and backward movement of the first tube clamp (421), the first tube clamp (421) and the second tube clamp (421, 521) provided to simultaneously clamp the first tubing (131) and the second tubing (131, 132) such that a central longitudinal axis of the first tubing (131) is parallel with a central longitudinal axis of the second tubing (131, 132);
heating elements (611, 911);
a carriage (24, 30) for supporting and moving a cutting wafer (95) thereon, the carriage (24, 30) being longitudinally moveable forward and backward and vertically moveable upward and downward;
a lead screw (26, 27) rotated by a motor (27), the lead screw (26, 27) having a lead screw nut (324) fixedly connected to the carriage (24, 30) wherein movement of the carriage (24, 30) is driven by rotation of the lead screw (26, 27);
wherein the carriage (24, 30) is moveable when supporting the wafer (95) thereon to bring the wafer (95) into contact with the heating elements (611, 911) to heat the wafer (95) to a pre-determined temperature,
wherein movement of the carriage (24, 30) forward and upward after heating of the wafer (95) moves the wafer (95) away from the heating elements (611, 911) and passes the wafer (95) through the first tubing (131) and the second tubing (131, 132) between the first tube clamp (421) and the second tube clamp (421, 521) thereby cutting the first tubing (131) and the second tubing (131, 132),
wherein the carriage (24, 30) is automatically mechanically connected to the first tube clamp (421) after the first tubing (131) and the second tubing (131, 132) have been cut such that the first tube clamp (421) moves forward together with the carriage (24, 30) when the carriage (24, 30) moves forward after the first tubing (131) and the second tubing (131, 132) have been cut, wherein when the first tube clamp (421) has been moved forward, a retained length (134, 135) of the first tubing (131) clamped by the first tube clamp (421) becomes axially aligned with a retained length (134, 135) of the second tubing (131, 132) clamped by the second tube clamp (421, 521);
wherein the device (10) is further configured to automatically and mechanically displace the second tube clamp (421, 521) laterally towards the first tube clamp (421) when the carriage (24, 30) moves forward after the retained length (134, 135) of the first tubing (131) has been axially aligned with the retained length (134, 135) of the second tubing (131, 132) and the wafer (95) has been removed from contact with the first tubing (131) and the second tubing (131, 132), thereby bringing together melted cut ends of the retained length (134, 135) of the first tubing (131) and the retained length (134, 135) of the second tubing (131, 132) to form a weld between the melted cut ends, thus forming a single length of tubing from the retained length (134, 135) of the first tubing (131) and the retained length (134, 135) of the second tubing (131, 132); and
an electrical control circuit board (17) programmed to control rotation of the motor (27) to drive movement of the carriage (24, 30).

2. The sterile tube connecting device (10) of claim 1, wherein the carriage (24, 30) comprises an upper carriage assembly (31, 32) and a lower carriage assembly (31, 32), the upper carriage assembly (31, 32) being in vertically oriented sliding engagement with the lower carriage assembly (31, 32), wherein both the upper and lower carriage assemblies (31, 32) are together moveable forward and backward and only the upper carriage assembly (31, 32) is moveable upward and downward.

3. The sterile tube connecting device (10) of claim 2, wherein the device (10) further comprises upstanding first and second cam plates (41, 51) provided one on each side of a path of travel of the carriage (24, 30), the first and second cam plates (41, 51) each having a cam track (411, 511) facing the path of travel, the upper carriage assembly (31, 32) having first and second upper carriage cam followers (18, 318) configured to rotatably engage the cam tracks (411, 511) of the first and second cam plates (41, 51) respectively, wherein longitudinal and vertical movement of the upper carriage assembly (31, 32) is guided by profiles of the cam tracks (411, 511), and/or wherein the upper carriage assembly (31, 32) comprises an upper carriage block (31, 311), a longitudinally oriented wafer support slot (301) provided on the upper carriage block (31, 311) for supporting the wafer (95) therein, wafer (95) guide pins (313, 314, 432) provided beside the wafer support slot (301) to guide movement and positioning of the wafer (95) in the wafer support slot (301), and a wafer latch (315) provided to secure the wafer (95) in the wafer support slot (301) and prevent backward movement of the wafer (95), and/or wherein the first tube clamp (421) is fixedly provided on top of a displacement mechanism assembly (42) that is longitudinally moveable forward and backward, the device (10) further comprising a displacement lock mechanism (43) to lock and unlock the displacement mechanism assembly (42) to prevent and allow longitudinal displacement of the first tube clamp (421) respectively.

4. The sterile tube connecting device (10) of claim 3, wherein the displacement lock mechanism (43) comprises a vertically moveable and longitudinally immoveable displacement lock cam guide (431), a displacement lock pin (432) having a free upper end and a lower end fixedly attached to the displacement lock cam guide (431), a displacement lock spring (433) that biases the displacement lock cam guide (431) and the displacement lock pin (432) upward, and a front pin slot (423) and a back pin (313, 314, 432) slot both provided on the displacement mechanism assembly (42) to separately engage the free upper end of the displacement lock pin (432) at different times to prevent longitudinal displacement of the first tube clamp (421); wherein the device (10) further comprises a pin (313, 314, 432) provided on the lower carriage assembly (31, 32) to engage the lock cam guide in order to lock the displacement mechanism assembly (42).

5. The sterile tube connecting device (10) of claim 4, wherein the cam tracks (411, 511) each comprise a first horizontal vector for horizontal movement of the upper carriage assembly (31, 32) to move the carriage (24, 30) to the heating elements (611, 911).

6. The sterile tube connecting device (10) of any one of claims 3 to 5, wherein the cam tracks (411, 511) each comprise a first angled ramp that is angled upward in the forward direction (11); wherein when the upper carriage cam followers (18, 318) engage the first angled ramp and the carriage (24, 30) is moved forward, the upper carriage assembly (31, 32) moves forward and upward along the first angled ramp for the wafer (95) supported on the upper carriage assembly (31, 32) to cut through the first tubing (131) and the second tubing (131, 132) while the displacement mechanism assembly (42) is locked by the displacement lock mechanism (43) to prevent longitudinal displacement of the first tube clamp (421) while the wafer (95) cuts the first tubing (131) and the second tubing (131, 132), and/or further comprising a displacement catch (312) and a displacement notch (425), wherein the displacement catch (312) is provided on one of the upper carriage assembly (31, 32) and the displacement mechanism assembly (42), and wherein the displacement notch (425) is provided on the other of the upper carriage assembly (31, 32) and the displacement mechanism, wherein the displacement catch (312) automatically engages the displacement notch (425) after the first and second tubings (131, 132) have been cut and the displacement lock mechanism (43) has unlocked the displacement mechanism assembly (42) to allow longitudinal movement of the first tube clamp (421), wherein engagement of the displacement catch (312) with the displacement notch (425) mechanically connects the upper carriage assembly (31, 32) with the first tube clamp (421) to move the first tube clamp (421) forward when the carriage (24, 30) moves forward after the first tubing (131) and the second tubing (131, 132) have been cut so as to axially align the retained length (134, 135) of the first tubing (131) with the retained length (134, 135) of the second tubing (131, 132).

7. The sterile tube connecting device (10) of claim 6, wherein the cam tracks (411, 511) each comprise a second horizontal vector for forward movement of the carriage assembly (31, 32) after the first tubing (131) and the second tubing (131, 132) have been cut.

8. The sterile tube connecting device (10) of any one of claims 3 to7, wherein the cam tracks (411, 511) each comprise a second angled ramp that is angled downward in the forward direction (11); wherein when the upper carriage cam followers (18, 318) engage the second angled ramp and the carriage (24, 30) is moved forward, the upper carriage assembly (31, 32) moves forward and downward to remove the wafer (95) supported on the upper carriage assembly (31, 32) from the cut first tubing (131) and the cut second tubing, and/or wherein the second tube clamp (421, 521) is fixedly provided on the second cam plate (41, 51), wherein the second cam plate (41, 51) is laterally displaceable leftward and rightward and biased towards the first cam plate (41) by welding mechanism springs (525) provided between the second cam plate (41, 51) and an immovable welding mechanism back plate, wherein the second cam plate (41, 51) comprises a welding cam guide (24, 524) facing the path of travel of the carriage (24, 30), wherein the lower carriage assembly (31, 32) comprises a lower carriage (24, 30) cam follower that rotatably engages the welding cam guide (24, 524), wherein the welding cam guide (24, 524) has a step (524) extending laterally outward such that when the lower carriage (24, 30) cam follower travels over the step (524), the welding mechanism springs (525) displace the second cam plate (41, 51) towards the first cam plate (41), thereby displacing the second tube clamp (421, 521) towards the first tube clamp (421) to bring together the melted cut ends of the retained length (134, 135) of the first tubing (131) and the retained length (134, 135) of the second tubing (131, 132).

9. The sterile tube connecting device (10) of any one of the preceding claims, further comprising a loading mechanism (70) configured to load the wafer (95) onto the carriage (24, 30) when the carriage (24, 30) moves backward from a heating area (954) to a loading area, the loading mechanism (70) comprising: a loading lower rack moveable backward from a forward position to a backward position by backward movement of the carriage (24, 30) when the carriage (24, 30) is in contact with a forward end of the loading lower rack; a loading pinion gear (72) in engagement with the loading lower rack; a loading upper rack in engagement with the pinion gear such that the loading upper rack moves in an opposite direction from movement of the loading lower rack; and a loading block (713) fixedly attached to the loading upper rack to push a wafer (95) forward out of a wafer cartridge (90) onto the carriage (24, 30) when the loading upper rack moves forward as a result of the loading lower rack moving backward when the carriage (24, 30) moves backward to the loading position.

10. The sterile tube connecting device (10) of claim 9, wherein the loading mechanism (70) further comprises a loading lower rack spring provided about the loading lower rack between a loading lower rack plunger head provided at the forward end of the loading lower rack and a loading mechanism housing (71) supporting the loading lower rack, wherein the loading lower rack spring returns the loading lower rack from the backward position to the forward position when the carriage (24, 30) moves forward away from the loading position.

11. The sterile tube connecting device (10) of any one of the preceding claims, further comprising an extraction mechanism (80) configured to remove the wafer (95) from the carriage (24, 30), the extraction mechanism (80) comprising: an extraction lower rack moveable forward from a backward position to a forward position by forward movement of the carriage (24, 30) when the carriage (24, 30) is in contact with a backward end of the extraction lower rack; an extraction pinion gear (83) in engagement with the extraction lower rack; an extraction upper rack in engagement with the pinion gear such that the extraction upper rack moves in an opposite direction from movement of the extraction lower rack; an extraction linkage (822) fixedly attached to the extraction upper rack; and an extraction catch moveable connected to the extraction linkage (822) and laterally displaceable leftward and rightward relative to the extraction linkage (822); the extraction catch (823) engaging a rear edge (97) of a used wafer (95) supported on the carriage (24, 30) when the extraction upper rack moves backward as a result of the extraction lower rack moving forward when the carriage (24, 30) moves forward to the extraction position after the first tubing (131) and the second tubing (131, 132) have been cut; wherein the wafer (95) is pulled forward off the carriage (24, 30) by the extraction catch (823) when the carriage (24, 30) moves backward away from the extraction position and the extraction lower rack is returned to the backward position by an extraction spring provided between an extraction lower rack plunger head provided at the backward end of the extraction lower rack and an upstanding front plate (22) of the device (10).

12. The sterile tube connecting device (10) of claim 11 when dependent on claim 9 or 10, wherein the device (10) further comprises a locating pin (322) provided on the carriage (24, 30) to interface with: a heating position sensor (442) provided at the heating area (954), a loading position sensor (441) provided at the loading area, and an extraction position sensor (443) provided at the extraction area, in order to automatically detect arrival of the carriage (24, 30) at the heating area (954), the loading area and the extraction area respectively.

13. The sterile tube connecting device (10) of claim 12, wherein in one cycle of use of the device (10), the electrical control circuit board (17) is programmed to control rotation of the motor (27) to move the carriage (24, 30) from the heating position backward to the loading position to load the wafer (95) onto the carriage (24, 30), from the loading position forward to the heating position to heat the wafer (95), from the heating position forward to the extraction position to cut and join the first tubing (131) and the second tubing (131, 132), and from the extraction position backward to the heating position to remove the wafer (95) from the carriage (24, 30).

14. A cutting wafer (95) for use with the sterile tube connecting device (10) of any one of claims 1 to 13, the cutting wafer (95) comprising a wafer working area (953) of a single layer of a metallic material provided to be heated to the pre-determined temperature to cut through the first tubing (131) and the second tubing (131, 132), a wafer heating area (954) comprising multiple layers of metallic material provided for receiving heat from the heating elements (611, 911) and to supply heat to the wafer working area (953).

15. The cutting wafer (95) of claim 14, wherein the wafer heating area (954) comprises two layers of the metallic material of the wafer working area (953), the two layers of the metallic material connected by a fold, and/or when dependent on claim 3, further comprising a wafer latch slot (956) for engagement by the wafer latch (315) to secure the wafer (95) in the wafer support slot (301) and prevent backward movement of the wafer (95).

## Patentansprüche

1. Sterile Schlauchverbindungsvorrichtung (10) zum Verbinden eines ersten Schlauchs (131) mit einem zweiten Schlauch (131, 132), wobei die Vorrichtung (10) Folgendes umfasst:
eine erste Schlauchklemme (421), die in Längsrichtung vorwärts und rückwärts beweglich ist;
eine zweite Schlauchklemme (421, 521), die von der ersten Schlauchklemme (421) beabstandet ist und seitlich nach links und rechts senkrecht zur Vorwärts- und Rückwärtsbewegung der ersten Schlauchklemme (421) beweglich ist, wobei die erste Schlauchklemme (421) und die zweite Schlauchklemme (421, 521) vorgesehen sind, um gleichzeitig den ersten Schlauch (131) und den zweiten Schlauch (131, 132) zu klemmen, so dass eine zentrale Längsachse des ersten Schlauchs (131) parallel zu einer zentralen Längsachse des zweiten Schlauchs (131, 132) ist;
Heizelemente (611, 911);
einen Schlitten (24, 30) zum Tragen und Bewegen eines Schneidwafers (95) darauf, wobei der Schlitten (24, 30) in Längsrichtung vorwärts und rückwärts beweglich ist und vertikal nach oben und unten beweglich ist;
eine Leitspindel (26, 27), die von einem Motor (27) gedreht wird, wobei die Leitspindel (26, 27) eine Leitspindelmutter (324) aufweist, die fest mit dem Schlitten (24, 30) verbunden ist, wobei die Bewegung des Schlittens (24, 30) durch die Drehung der Leitspindel (26, 27) angetrieben wird;
wobei der Schlitten (24, 30) beweglich ist, wenn der Wafer (95) darauf getragen wird, um den Wafer (95) in Kontakt mit den Heizelementen (611, 911) zu bringen, um den Wafer (95) auf eine vorbestimmte Temperatur zu erwärmen,
wobei die Bewegung des Schlittens (24, 30) nach vorne und oben nach dem Erwärmen des Wafers (95) den Wafer (95) von den Heizelementen (611, 911) weg bewegt und den Wafer (95) durch den ersten Schlauch (131) und den zweiten Schlauch (131, 132) zwischen der ersten Schlauchklemme (421) und der zweiten Schlauchklemme (421, 521) führt, wodurch der erste Schlauch (131) und der zweite Schlauch (131, 132) geschnitten werden,
wobei der Schlitten (24, 30) automatisch mechanisch mit der ersten Schlauchklemme (421) verbunden wird, nachdem der erste Schlauch (131) und der zweite Schlauch (131, 132) geschnitten wurden, so dass sich die erste Schlauchklemme (421) zusammen mit dem Schlitten (24, 30) vorwärts bewegt, wenn sich der Schlitten (24, 30) vorwärts bewegt, nachdem der erste Schlauch (131) und der zweite Schlauch (131, 132) geschnitten wurden, wobei, wenn die erste Schlauchklemme (421) vorwärts bewegt wurde, eine gehaltene Länge (134, 135) des ersten Schlauchs (131), der von der ersten Schlauchklemme (421) geklemmt wird, axial mit einer gehaltenen Länge (134, 135) des zweiten Schlauchs (131, 132), der von der zweiten Schlauchklemme (421, 521) geklemmt wird, ausgerichtet wird;
wobei die Vorrichtung (10) ferner konfiguriert ist, um automatisch und mechanisch die zweite Schlauchklemme (421, 521) seitlich zur ersten Schlauchklemme (421) zu verschieben, wenn sich der Schlitten (24, 30) vorwärts bewegt, nachdem die gehaltene Länge (134, 135) des ersten Schlauchs (131) axial mit der gehaltenen Länge (134, 135) des zweiten Schlauchs (131, 132) ausgerichtet wurde und der Wafer (95) aus dem Kontakt mit dem ersten Schlauch (131) und dem zweiten Schlauch (131, 132) entfernt wurde, wodurch geschmolzene Schnittenden der gehaltenen Länge (134, 135) des ersten Schlauchs (131) und der gehaltenen Länge (134, 135) des zweiten Schlauchs (131, 132) zusammengebracht werden, um eine Schweißnaht zwischen den geschmolzenen Schnittenden zu bilden, wodurch eine einzige Schlauchlänge aus der gehaltenen Länge (134, 135) des ersten Schlauchs (131) und der gehaltenen Länge (134, 135) des zweiten Schlauchs (131, 132) gebildet wird; und
eine elektrische Steuerplatine (17), die programmiert ist, um die Drehung des Motors (27) zu steuern, um die Bewegung des Schlittens (24, 30) anzutreiben.

2. Sterile Schlauchverbindungsvorrichtung (10) nach Anspruch 1, wobei der Schlitten (24, 30) eine obere Schlittenanordnung (31, 32) und eine untere Schlittenanordnung (31, 32) umfasst, wobei die obere Schlittenanordnung (31, 32) in vertikal ausgerichtetem Gleiteingriff mit der unteren Schlittenanordnung (31, 32) steht, wobei sowohl die obere als auch die untere Schlittenanordnung (31, 32) zusammen vorwärts und rückwärts beweglich sind und nur die obere Schlittenanordnung (31, 32) nach oben und unten beweglich ist.

3. Sterile Schlauchverbindungsvorrichtung (10) nach Anspruch 2, wobei die Vorrichtung (10) ferner eine aufrechte erste und zweite Nockenplatte (41, 51) umfasst, die auf jeder Seite eines Bewegungswegs des Schlittens (24, 30) vorgesehen sind, wobei die erste und zweite Nockenplatte (41, 51) jeweils eine Nockenbahn (411, 511) aufweisen, die dem Bewegungsweg zugewandt ist, wobei die obere Schlittenanordnung (31, 32) einen ersten und zweiten oberen Schlittennockenstößel (18, 318) aufweist, die konfiguriert sind, um drehbar in die Nockenbahnen (411, 511) der ersten bzw. zweiten Nockenplatte (41, 51) einzugreifen, wobei die Längs- und Vertikalbewegung der oberen Schlittenanordnung (31, 32) durch Profile der Nockenbahnen (411, 511) geführt wird, und/oder wobei die obere Schlittenanordnung (31, 32) einen oberen Schlittenblock (31, 311), einen in Längsrichtung ausgerichteten Waferträgerschlitz (301), der auf dem oberen Schlittenblock (31, 311) zum Tragen des Wafers (95) darin vorgesehen ist, Wafer (95)-Führungsstifte (313, 314, 432), die neben dem Waferträgerschlitz (301) vorgesehen sind, um die Bewegung und Positionierung des Wafers (95) im Waferträgerschlitz (301) zu führen, und eine Waferverriegelung (315), die vorgesehen ist, um den Wafer (95) im Waferträgerschlitz (301) zu sichern und die Rückwärtsbewegung des Wafers (95) zu verhindern, umfasst, und/oder wobei die erste Schlauchklemme (421) fest auf einer Verschiebemechanismusanordnung (42) vorgesehen ist, die in Längsrichtung vorwärts und rückwärts beweglich ist, wobei die Vorrichtung (10) ferner einen Verschiebeverriegelungsmechanismus (43) zum Verriegeln und Entriegeln der Verschiebemechanismusanordnung (42) umfasst, um die Längsverschiebung der ersten Schlauchklemme (421) zu verhindern bzw. zu ermöglichen.

4. Sterile Schlauchverbindungsvorrichtung (10) nach Anspruch 3, wobei der Verschiebeverriegelungsmechanismus (43) eine vertikal bewegliche und in Längsrichtung unbewegliche Verschiebeverriegelungsnockenführung (431), einen Verschiebeverriegelungsstift (432) mit einem freien oberen Ende und einem unteren Ende, die fest an der Verschiebeverriegelungsnockenführung (431) angebracht sind, eine Verschiebeverriegelungsfeder (433), die die Verschiebeverriegelungsnockenführung (431) und den Verschiebeverriegelungsstift (432) nach oben vorspannt, und einen vorderen Stiftschlitz (423) und einen hinteren Stiftschlitz (313, 314, 432), die beide auf der Verschiebemechanismusanordnung (42) vorgesehen sind, um separat in das freie obere Ende des Verschiebeverriegelungsstifts (432) zu unterschiedlichen Zeiten einzugreifen, um die Längsverschiebung der ersten Schlauchklemme (421) zu verhindern, umfasst; wobei die Vorrichtung (10) ferner einen Stift (313, 314, 432) umfasst, der auf der unteren Schlittenanordnung (31, 32) vorgesehen ist, um in die Verriegelungsnockenführung einzugreifen, um die Verschiebemechanismusanordnung (42) zu verriegeln.

5. Sterile Schlauchverbindungsvorrichtung (10) nach Anspruch 4, wobei die Nockenbahnen (411, 511) jeweils einen ersten horizontalen Vektor für die horizontale Bewegung der oberen Schlittenanordnung (31, 32) umfassen, um den Schlitten (24, 30) zu den Heizelementen (611, 911) zu bewegen.

6. Sterile Schlauchverbindungsvorrichtung (10) nach einem der Ansprüche 3 bis 5, wobei die Nockenbahnen (411, 511) jeweils eine erste abgewinkelte Rampe umfassen, die in der Vorwärtsrichtung (11) nach oben abgewinkelt ist; wobei, wenn die oberen Schlittennockenfolger (18, 318) in die erste abgewinkelte Rampe eingreifen und der Schlitten (24, 30) vorwärts bewegt wird, sich die obere Schlittenanordnung (31, 32) entlang der ersten abgewinkelten Rampe vorwärts und nach oben bewegt, damit der Wafer (95), der auf der oberen Schlittenanordnung (31, 32) getragen wird, durch den ersten Schlauch (131) und den zweiten Schlauch (131, 132) schneidet, während die Verschiebemechanismusanordnung (42) durch den Verschiebeverriegelungsmechanismus (43) verriegelt ist, um die Längsverschiebung der ersten Schlauchklemme (421) zu verhindern, während der Wafer (95) den ersten Schlauch (131) und den zweiten Schlauch (131, 132) schneidet, und/oder ferner umfassend eine Verschiebearretierung (312) und eine Verschiebekerbe (425), wobei die Verschiebearretierung (312) auf einer der oberen Schlittenanordnung (31, 32) und der Verschiebemechanismusanordnung (42) vorgesehen ist, und wobei die Verschiebekerbe (425) auf der anderen der oberen Schlittenanordnung (31, 32) und dem Verschiebemechanismus vorgesehen ist, wobei die Verschiebearretierung (312) automatisch in die Verschiebekerbe (425) eingreift, nachdem der erste und der zweite Schlauch (131, 132) geschnitten wurden und der Verschiebeverriegelungsmechanismus (43) die Verschiebemechanismusanordnung (42) entriegelt hat, um die Längsbewegung der ersten Schlauchklemme (421) zu ermöglichen, wobei der Eingriff der Verschiebearretierung (312) mit der Verschiebekerbe (425) die obere Schlittenanordnung (31, 32) mechanisch mit der ersten Schlauchklemme (421) verbindet, um die erste Schlauchklemme (421) vorwärts zu bewegen, wenn sich der Schlitten (24, 30) vorwärts bewegt, nachdem der erste Schlauch (131) und der zweite Schlauch (131, 132) geschnitten wurden, um die gehaltene Länge (134, 135) des ersten Schlauchs (131) axial mit der gehaltenen Länge (134, 135) des zweiten Schlauchs (131, 132) auszurichten.

7. Sterile Schlauchverbindungsvorrichtung (10) nach Anspruch 6, wobei die Nockenbahnen (411, 511) jeweils einen zweiten horizontalen Vektor für die Vorwärtsbewegung der Schlittenanordnung (31, 32) umfassen, nachdem der erste Schlauch (131) und der zweite Schlauch (131, 132) geschnitten wurden.

8. Sterile Schlauchverbindungsvorrichtung (10) nach einem der Ansprüche 3 bis 7, wobei die Nockenbahnen (411, 511) jeweils eine zweite abgewinkelte Rampe umfassen, die in der Vorwärtsrichtung (11) nach unten abgewinkelt ist; wobei, wenn die oberen Schlittennockenfolger (18, 318) in die zweite abgewinkelte Rampe eingreifen und der Schlitten (24, 30) vorwärts bewegt wird, sich die obere Schlittenanordnung (31, 32) vorwärts und nach unten bewegt, um den Wafer (95), der auf der oberen Schlittenanordnung (31, 32) getragen wird, von dem geschnittenen ersten Schlauch (131) und dem geschnittenen zweiten Schlauch zu entfernen, und/oder wobei die zweite Schlauchklemme (421, 521) fest auf der zweiten Nockenplatte (41, 51) vorgesehen ist, wobei die zweite Nockenplatte (41, 51) seitlich nach links und rechts verschiebbar ist und in Richtung der ersten Nockenplatte (41) durch Schweißmechanismusfedern (525) vorgespannt ist, die zwischen der zweiten Nockenplatte (41, 51) und einer unbeweglichen Schweißmechanismusrückplatte vorgesehen sind, wobei die zweite Nockenplatte (41, 51) eine Schweißnockenführung (24, 524) umfasst, die dem Bewegungspfad des Schlittens (24, 30) zugewandt ist, wobei die untere Schlittenanordnung (31, 32) einen unteren Schlittennockenfolger (24, 30) umfasst, der drehbar in die Schweißnockenführung (24, 524) eingreift, wobei die Schweißnockenführung (24, 524) eine Stufe (524) aufweist, die sich seitlich nach außen erstreckt, so dass, wenn sich der untere Schlittennockenfolger (24, 30) über die Stufe (524) bewegt, die Schweißmechanismusfedern (525) die zweite Nockenplatte (41, 51) in Richtung der ersten Nockenplatte (41) verschieben, wodurch die zweite Schlauchklemme (421, 521) in Richtung der ersten Schlauchklemme (421) verschoben wird, um die geschmolzenen geschnittenen Enden der gehaltenen Länge (134, 135) des ersten Schlauchs (131) und der gehaltenen Länge (134, 135) des zweiten Schlauchs (131, 132) zusammenzuführen.

9. Sterile Schlauchverbindungsvorrichtung (10) nach einem der vorhergehenden Ansprüche, ferner umfassend einen Lademechanismus (70), der konfiguriert ist, um den Wafer (95) auf den Schlitten (24, 30) zu laden, wenn sich der Schlitten (24, 30) von einem Heizbereich (954) zu einem Ladebereich rückwärts bewegt, wobei der Lademechanismus (70) Folgendes umfasst: ein unteres Ladegestell, das durch Rückwärtsbewegung des Schlittens (24, 30) von einer Vorwärtsposition zu einer Rückwärtsposition rückwärts beweglich ist, wenn der Schlitten (24, 30) in Kontakt mit einem vorderen Ende des unteren Ladegestells steht; ein Laderitzel (72) in Eingriff mit dem unteren Ladegestell; ein oberes Ladegestell in Eingriff mit dem Ritzel, so dass sich das obere Ladegestell in einer entgegengesetzten Richtung zur Bewegung des unteren Ladegestells bewegt; und einen Ladeblock (713), der fest an dem oberen Ladegestell angebracht ist, um einen Wafer (95) vorwärts aus einer Waferkartusche (90) auf den Schlitten (24, 30) zu schieben, wenn sich das obere Ladegestell als Folge der Rückwärtsbewegung des unteren Ladegestells vorwärts bewegt, wenn sich der Schlitten (24, 30) rückwärts in die Ladeposition bewegt.

10. Sterile Schlauchverbindungsvorrichtung (10) nach Anspruch 9, wobei der Lademechanismus (70) ferner eine untere Ladegestellfeder umfasst, die um das untere Ladegestell zwischen einem unteren Ladegestellkolbenkopf, der am vorderen Ende des unteren Ladegestells vorgesehen ist, und einem Lademechanismusgehäuse (71), das das untere Ladegestell trägt, vorgesehen ist, wobei die untere Ladegestellfeder das untere Ladegestell von der Rückwärtsposition in die Vorwärtsposition zurückführt, wenn sich der Schlitten (24, 30) vorwärts von der Ladeposition weg bewegt.

11. Sterile Schlauchverbindungsvorrichtung (10) nach einem der vorhergehenden Ansprüche, ferner umfassend einen Extraktionsmechanismus (80), der konfiguriert ist, um den Wafer (95) von dem Schlitten (24, 30) zu entfernen, wobei der Extraktionsmechanismus (80) Folgendes umfasst: ein unteres Extraktionsgestell, das durch Vorwärtsbewegung des Schlittens (24, 30) von einer Rückwärtsposition zu einer Vorwärtsposition vorwärts beweglich ist, wenn der Schlitten (24, 30) in Kontakt mit einem hinteren Ende des unteren Extraktionsgestells steht; ein Extraktionsritzel (83) in Eingriff mit dem unteren Extraktionsgestell; ein oberes Extraktionsgestell in Eingriff mit dem Ritzel, so dass sich das obere Extraktionsgestell in einer entgegengesetzten Richtung zur Bewegung des unteren Extraktionsgestells bewegt; ein Extraktionsgestänge (822), das fest an dem oberen Extraktionsgestell angebracht ist; und eine Extraktionsklinke, die beweglich mit dem Extraktionsgestänge (822) verbunden ist und seitlich nach links und rechts relativ zum Extraktionsgestänge (822) verschiebbar ist; wobei die Extraktionsklinke (823) mit einer Hinterkante (97) eines verwendeten Wafers (95) in Eingriff steht, der auf dem Schlitten (24, 30) getragen wird, wenn sich das obere Extraktionsgestell als Folge der Vorwärtsbewegung des unteren Extraktionsgestells rückwärts bewegt, wenn sich der Schlitten (24, 30) vorwärts in die Extraktionsposition bewegt, nachdem der erste Schlauch (131) und der zweite Schlauch (131, 132) geschnitten wurden; wobei der Wafer (95) durch die Extraktionsklinke (823) vom Schlitten (24, 30) vorwärts abgezogen wird, wenn sich der Schlitten (24, 30) rückwärts von der Extraktionsposition weg bewegt, und das untere Extraktionsgestell durch eine Extraktionsfeder, die zwischen einem unteren Extraktionsgestellkolbenkopf, der am hinteren Ende des unteren Extraktionsgestells vorgesehen ist, und einer aufrechten Frontplatte (22) der Vorrichtung (10) vorgesehen ist, in die Rückwärtsposition zurückgeführt wird.

12. Sterile Schlauchverbindungsvorrichtung (10) nach Anspruch 11, wenn abhängig von Anspruch 9 oder 10, wobei die Vorrichtung (10) ferner einen Positionierungsstift (322) umfasst, der auf dem Schlitten (24, 30) vorgesehen ist, um mit Folgendem verbunden zu sein: einem Heizpositionssensor (442), der am Heizbereich (954) vorgesehen ist, einem Ladepositionssensor (441), der am Ladebereich vorgesehen ist, und einem Extraktionspositionssensor (443), der am Extraktionsbereich vorgesehen ist, um automatisch die Ankunft des Schlittens (24, 30) am Heizbereich (954), am Ladebereich bzw. am Extraktionsbereich zu erfassen.

13. Sterile Schlauchverbindungsvorrichtung (10) nach Anspruch 12, wobei in einem Verwendungszyklus der Vorrichtung (10) die elektrische Steuerplatine (17) programmiert ist, um die Drehung des Motors (27) zu steuern, um den Schlitten (24, 30) von der Heizposition rückwärts in die Ladeposition zu bewegen, um den Wafer (95) auf den Schlitten (24, 30) zu laden, von der Ladeposition vorwärts in die Heizposition, um den Wafer (95) zu erwärmen, von der Heizposition vorwärts in die Extraktionsposition, um den ersten Schlauch (131) und den zweiten Schlauch (131, 132) zu schneiden und zu verbinden, und von der Extraktionsposition rückwärts in die Heizposition, um den Wafer (95) vom Schlitten (24, 30) zu entfernen.

14. Schneidwafer (95) zur Verwendung mit der sterilen Schlauchverbindungsvorrichtung (10) nach einem der Ansprüche 1 bis 13, wobei der Schneidwafer (95) einen Waferarbeitsbereich (953) aus einer einzelnen Schicht aus einem metallischen Material umfasst, der vorgesehen ist, um auf die vorbestimmte Temperatur erwärmt zu werden, um durch den ersten Schlauch (131) und den zweiten Schlauch (131, 132) zu schneiden, einen Waferheizbereich (954), der mehrere Schichten aus metallischem Material umfasst, die vorgesehen sind, um Wärme von den Heizelementen (611, 911) aufzunehmen und dem Waferarbeitsbereich (953) Wärme zuzuführen.

15. Schneidwafer (95) nach Anspruch 14, wobei der Waferheizbereich (954) zwei Schichten aus dem metallischen Material des Waferarbeitsbereichs (953) umfasst, wobei die zwei Schichten aus dem metallischen Material durch eine Falte verbunden sind, und/oder wenn abhängig von Anspruch 3, ferner umfassend einen Waferverriegelungsschlitz (956) zum Eingriff durch die Waferverriegelung (315), um den Wafer (95) im Waferträgerschlitz (301) zu sichern und eine Rückwärtsbewegung des Wafers (95) zu verhindern.

## Revendications

1. Un dispositif de connexion de tubes stériles (10) pour relier une première conduite (131) à une seconde conduite (131, 132), le dispositif (10) comprenant :
une première pince de tube (421) mobile longitudinalement vers l'avant et vers l'arrière ;
une seconde pince de tube (421, 521) espacée de la première pince de tube (421) et mobile latéralement vers la gauche et vers la droite perpendiculairement au mouvement vers l'avant et vers l'arrière de la première pince de tube (421), la première pince de tube (421) et la seconde pince de tube (421, 521) étant faites pour pincer simultanément la première conduite (131) et la seconde conduite (131, 132) de manière qu'un axe longitudinal central de la première conduite (131) soit parallèle à un axe central longitudinal de la seconde conduite (131, 132) ;
des éléments chauffants (611, 911) ;
un chariot (24, 30) destinée à supporter et à déplacer une plaquette de découpe (95) qui est dessus, le chariot (24, 30) étant mobile longitudinalement vers l'avant et vers l'arrière et mobile verticalement vers le haut et vers le bas ;
une vis-mère (26, 27) entraînée en rotation par un moteur (27), la vis-mère (26, 27) possédant un écrou de vis-mère (324) relié fixement au chariot (24, 30), le chariot (24, 30) étant entraîné en mouvement par la rotation de la vis-mère (26, 27) ;
dans lequel le chariot (24, 30) est mobile lorsqu'il supporte la plaquette (95) qui est dessus pour amener la plaquette (95) en contact avec les éléments chauffants (611, 911) pour chauffer la plaquette (95) à une température prédéterminée,
dans lequel le mouvement du chariot (24, 30) vers l'avant et vers le haut après chauffage de la plaquette (95) déplace la plaquette (95) en éloignement des éléments chauffants (611, 911) et fait que la plaquette (95) traverse la première conduite (131) et la seconde conduite (131, 132) entre la première pince de tube (421) et la seconde pince de tube (421, 521), en découpant ainsi la première conduite (131) et la seconde conduite (131, 132),
dans lequel le chariot (24, 30) est mécaniquement et automatiquement connecté à la première pince de tube (421) après que la première conduite (131) et la seconde conduite (131, 132) ont été découpées de sorte que la première pince de tube (421) se déplace vers l'avant en même temps que le chariot (24, 30) lorsque le chariot (24, 30) se déplace vers l'avant après que la première conduite (131) et la seconde conduite (131, 132) ont été découpées, dans lequel, lorsque la première pince de tube (421) a été déplacée vers l'avant, une longueur retenue (134, 135) de la première conduite (131) pincée par la première pince de tube (421) devient axialement alignée avec une longueur retenue (134, 135) de la seconde conduite (131, 132) pincée par la seconde pince de tube (421, 521) ;
dans lequel le dispositif (10) est en outre configuré pour déplacer mécaniquement et automatiquement la seconde pince de tube (421, 521) latéralement vers la première pince de tube (421) lorsque le chariot (24, 30) se déplace vers l'avant après que la longueur retenue (134, 135) de la première conduite (131) a été axialement alignée avec la longueur retenue (134, 135) de la seconde conduite (131, 132) et que la plaquette (95) a été retirée du contact avec la première conduite (131) et la seconde conduite (131, 132), amenant ainsi ensemble des extrémités découpées fondues de la longueur retenue (134, 135) de la première conduite (131) et de la longueur retenue (134, 135) de la seconde conduite (131, 132) pour former une soudure entre les extrémités découpées fondues, formant ainsi une longueur unique de conduite à partir de la longueur retenue (134, 135) de la première conduite (131) et de la longueur retenue (134, 135) de la seconde conduite (131, 132) ; et
une carte de circuit de contrôle électrique (17) programmée pour contrôler la rotation du moteur (27) pour entraîner en mouvement le chariot (24, 30).

2. Le dispositif de connexion de tubes stériles (10) de la revendication 1, dans lequel le chariot (24, 30) comprend un bloc de chariot supérieur (31, 32) et un bloc de chariot inférieur (31, 32), le bloc de chariot supérieur (31, 32) venant en prise à coulissement dans une orientation verticale avec le bloc de chariot inférieur (31, 32), dans lequel les blocs de chariot à la fois supérieur et inférieur (31, 32) sont mobiles ensemble vers l'avant et vers l'arrière et seul le bloc de chariot supérieur (31, 32) est mobile vers le haut et vers le bas.

3. Le dispositif de connexion de tubes stériles (10) de la revendication 2, dans lequel le dispositif (10) comprend une première et une seconde plaquette de came dressées (41, 51) disposées chacune sur un côté d'un trajet de déplacement du chariot (24, 30), la première et la seconde plaquette de came (41, 51) possédant chacune un chemin de came (411, 511) tourné vers le trajet de déplacement, le bloc de chariot supérieur (31, 32) possédant un premier et un second doigt de came de chariot supérieur (18, 78) configurés pour venir en prise à rotation avec les trajets de déplacement (411, 511) de la première et de la seconde plaquette de came (41, 51), respectivement, dans lequel le mouvement longitudinal et vertical du bloc de chariot supérieur (31, 32) est guidé par des profils des trajets de déplacement (411, 511) et/ou dans lequel le bloc de chariot supérieur (31, 32) comprend un bloc de chariot supérieur (31, 311), une fente support de plaquette orientée longitudinalement (301) située sur le bloc de chariot supérieur (31, 311) pour supporter la plaquette (95) qui est dedans, des broches de guidage (313, 314, 432) de la plaquette (95) disposées à côté de la fente support de plaquette (301) pour guider le mouvement et le positionnement de la plaquette (95) dans la fente de support de plaquette (301), et un verrou de plaquette (315) prévu pour assujettir la plaquette (95) dans la fente de support de plaquette (301) et empêcher un mouvement vers l'arrière de la plaquette (95), et/ou dans lequel la première pince de tube (421) est disposée fixe au-dessus d'un bloc de mécanisme de déplacement (42) qui est mobile longitudinalement vers l'avant et vers l'arrière, le dispositif (10) comprenant en outre un mécanisme de verrouillage de déplacement (43) pour verrouiller et déverrouiller le bloc de mécanisme de déplacement (42) pour respectivement empêcher et permettre un déplacement longitudinal de la première pince de tube (421).

4. Le dispositif de connexion de tubes stériles (10) de la revendication 3, dans lequel le mécanisme de verrouillage de déplacement (43) comprend un guide de came de verrouillage de déplacement mobile verticalement et immobile longitudinalement, une broche de verrouillage de déplacement (432) avec une extrémité supérieure libre et une extrémité inférieure assujettie fixement à la came de guidage de verrouillage de déplacement (431), un ressort de verrouillage de déplacement (433) qui sollicite le guide de came de verrouillage de déplacement (431) et la broche de verrouillage de déplacement (432) vers le haut, et une fente de broche avant (423) et une fente de broche arrière (313, 314, 432) toutes deux disposées sur le bloc de mécanisme de déplacement (42) pour venir séparément en prise avec l'extrémité supérieure libre de la broche de verrouillage de déplacement (432) à des moments différents pour empêcher le déplacement longitudinal de la première pince de tube (421) ;
dans lequel le dispositif (10) comprend en outre une broche (313, 314, 432) située sur le bloc de chariot inférieur (31, 32) pour venir en prise avec le guide de came de verrouillage afin de verrouiller le bloc de mécanisme de déplacement (42).

5. Le dispositif de connexion de tubes stériles (10) de la revendication 4, dans lequel les trajets de déplacement (411, 511) comprennent chacun un premier vecteur horizontal pour que le mouvement horizontal du bloc de chariot supérieur (31, 32) déplace le chariot (24, 30) vers les éléments chauffants (611, 911).

6. Le dispositif de connexion de tubes stériles (10) de l'une des revendications 3 à 5, dans lequel les trajets de déplacement (411, 511) comprennent chacun une première rampe inclinée qui fait un angle vers le haut dans la direction vers l'avant (11) ; dans lequel, lorsque les doigts de came du chariot supérieur (18, 318) viennent en prise avec la première rampe inclinée et que le chariot (24, 30) est déplacé vers l'avant, le bloc de chariot supérieur (31, 32) se déplace vers l'avant et vers le haut le long de la première rampe inclinée pour que la plaquette (95) portée par le bloc de chariot supérieur (31, 32) effectue une découpe au travers de la première conduite (131) et de la seconde conduite (131, 132) tandis que le bloc de mécanisme de déplacement (42) est verrouillé par le mécanisme de verrouillage de déplacement (43) pour empêcher un déplacement longitudinal de la première pince de tube (421) pendant que la plaquette (95) découpe la première conduite (131) et la seconde conduite (131, 132), et/ou comprenant en outre un cran de déplacement (312) et une encoche de déplacement (425), dans lequel le cran de déplacement (312) est situé sur l'un d'entre le bloc de chariot supérieur (31, 32) et le bloc de mécanisme de déplacement (42), et dans lequel l'encoche de déplacement (425) est disposée sur l'autre d'entre le bloc de chariot supérieur (31, 32) et le mécanisme de déplacement, dans lequel le cran de déplacement (312) vient automatiquement en prise avec l'encoche de déplacement (425) après que la première et la seconde conduite (131, 132) ont été découpées et que le mécanisme de verrouillage de déplacement (43) a déverrouillé le bloc de mécanisme de déplacement (42) pour permettre un mouvement longitudinal de la première pince de tube (421), dans lequel la venue en prise du cran de déplacement (312) avec l'encoche de déplacement (425) connecte mécaniquement le bloc de chariot supérieur (31, 32) à la première pince de tube (421) pour déplacer la première pince de tube (421) vers l'avant lorsque le chariot (24, 30) se déplace vers l'avant après que la première conduite (131) et la seconde conduite (131, 132) ont été découpées de manière à aligner axialement la longueur retenue (134, 135) de la première condition (131) avec la longueur retenue (134, 135) de la seconde conduite (131, 132).

7. Le dispositif de connexion de tubes stériles (10) de la revendication 6, dans lequel les trajets de déplacement (411, 511) comprennent chacun un second vecteur horizontal pour un mouvement vers l'avant du bloc de chariot (31, 32) après que la première conduite (131) et la seconde conduite (131, 132) ont été découpées.

8. Le dispositif de connexion de tubes stériles (10) de l'une des revendications 3 à 7, dans lequel les trajets de déplacement (411, 511) comprennent chacun une seconde rampe inclinée qui forme un angle vers le bas dans la direction vers l'avant (11) ; dans lequel, lorsque les doigts de came du chariot supérieur (18, 318) viennent en prise avec la seconde rampe inclinée et que le chariot (24, 30) est déplacé vers l'avant, le bloc de chariot supérieur (31, 32) se déplace vers l'avant et vers le bas pour retirer la plaquette (95) supportée sur le bloc de chariot supérieur (31, 32) d'avec la première conduite découpée (131) et la seconde conduite découpée, et/ou dans lequel la seconde pince de tube (421, 521) est disposée fixe sur la seconde plaque de came (41, 51), dans lequel la seconde plaque de came (41, 51) est déplaçable latéralement vers la gauche et vers la droite et est sollicitée en direction de la première plaque de came (41) par des ressorts de mécanisme de soudure (525) disposés entre la seconde plaque de came (41, 51) et une plaque arrière immobile de mécanisme de soudure, dans lequel la seconde plaque de came (41, 51) comprend un guide de came de soudure (24, 524) tourné vers le trajet de déplacement du chariot (24, 30), dans lequel le bloc de chariot inférieur (31, 32) comprend un doigt de came de chariot inférieur (24, 30) qui vient en prise à rotation avec le guide de came de soudure (24, 524), dans lequel le guide de came de soudure (24, 524) possède un gradin (524) s'étendant latéralement vers l'extérieur de telle sorte que, lorsque le doigt de came de chariot inférieur (24, 30) se déplace au-dessus du gradin (524), les ressorts de mécanisme de soudure (525) déplacent la seconde plaque de came (41, 51) en direction de la première plaque de came (41), déplaçant ainsi la seconde pince de tube (421, 521) en direction de la première pince de tube (421) pour réunir les extrémités découpées fondues de la longueur retenue (134, 135) de la première conduite (131) et de la longueur retenue (134, 135) de la seconde conduite (131, 132).

9. Le dispositif de connexion de tubes stériles (10) de l'une des revendications précédentes, comprenant en outre un mécanisme de chargement (70) configuré pour charger la plaquette (95) sur le chariot (24, 30) lorsque le chariot (24, 30) se déplace vers l'arrière d'une zone de chauffage (954) à une zone de chargement, le mécanisme de chargement (70) comprenant une crémaillère inférieure de chargement mobile vers l'arrière depuis une position vers l'avant vers une position vers l'arrière par un mouvement vers l'arrière du chariot (24, 30) lorsque le chariot (24, 30) est en contact avec une extrémité avant de la crémaillère inférieure de chargement ; un pignon de chargement (72) en prise avec la crémaillère inférieure de chargement ; une crémaillère supérieure de chargement en prise avec le pignon de telle sorte que la crémaillère supérieure de chargement se déplace dans une direction opposée à celle du déplacement de la crémaillère inférieure de chargement ; et un bloc de chargement (713) monté fixe sur la crémaillère supérieure de chargement pour venir pousser vers l'avant une plaquette (95) hors d'une cartouche de plaquette (90) jusque sur le chariot (24, 30) lorsque la crémaillère supérieure de chargement se déplace vers l'avant du fait que la crémaillère inférieure de chargement se déplace vers l'arrière lorsque le chariot (24, 30) se déplace vers l'arrière vers la position de chargement.

10. Le dispositif de connexion de tubes stériles (10) de la revendication 9, dans lequel le mécanisme de chargement (70) comprend en outre un ressort de crémaillère inférieure de chargement situé autour de la crémaillère inférieure de chargement entre une tête de plongeur de crémaillère inférieure de chargement située à l'extrémité avant de la crémaillère inférieure de chargement et un boîtier de mécanisme de chargement (71) supportant la crémaillère inférieure de chargement, dans lequel le ressort de crémaillère inférieure de chargement ramène la crémaillère inférieure de chargement de la position vers l'arrière à la position vers l'avant lorsque le chariot (24, 30) se déplace vers l'avant en éloignement de la position de chargement.

11. Le dispositif de connexion de tubes stériles (10) de l'une des revendications précédentes, comprenant en outre un mécanisme d'extraction (80) configuré pour retirer la plaquette (95) du chariot (24, 30), le mécanisme d'extraction (80) comprenant : une crémaillère inférieure d'extraction mobile vers l'avant d'une position vers l'arrière à une position vers l'avant par un mouvement vers l'avant du chariot (24, 30) lorsque le chariot (24, 30) est en contact avec une extrémité arrière de la crémaillère inférieure d'extraction ; un pignon d'extraction (83) en prise avec la crémaillère inférieure d'extraction ; une crémaillère supérieure d'extraction en prise avec le pignon de telle manière que la crémaillère supérieure d'extraction se déplace dans un sens opposé de celui du mouvement de la crémaillère inférieure d'extraction ; une articulation d'extraction (822) montée fixe sur la crémaillère supérieure d'extraction ; et un cran d'extraction relié de façon mobile à l'articulation d'extraction (822) et déplaçable latéralement vers la gauche et vers la droite par rapport à l'articulation d'extraction (822) ; le cran d'extraction (823) venant en prise avec un bord arrière (97) d'une plaquette utilisée (95) supportée sur le chariot (24, 30) lorsque la crémaillère supérieure d'extraction se déplace vers l'arrière du fait de la crémaillère inférieure d'extraction qui se déplace vers l'avant lorsque le chariot (24, 30) se déplace vers l'avant vers la position d'extraction après que la première conduite (131) et la seconde conduite (131, 132) ont été découpées ; dans lequel la plaquette (95) est tirée vers l'avant hors du chariot (24, 30) par le cran d'extraction (823) lorsque le chariot (24, 30) se déplace vers l'arrière en éloignement de la position d'extraction et que la crémaillère inférieure d'extraction est ramenée à la position arrière par un ressort d'extraction situé entre une tête de plongeur de crémaillère inférieure d'extraction située à l'extrémité arrière de la crémaillère inférieure d'extraction et une plaque avant dressée (22) du dispositif (10).

12. Le dispositif de connexion de tubes stériles (10) de la revendication 11 prise en dépendance de la revendication 9 ou 10, dans lequel le dispositif (10) comprend en outre une broche repère (322) montée sur le chariot (24, 30) pour s'interfacer avec : un capteur de position de chauffage (442) situé au niveau de la zone de chauffage (954), un capteur de position de chargement (441) situé au niveau de la zone de chargement, et un capteur de position d'extraction (443) situé au niveau de la zone d'extraction, afin de détecter automatiquement l'arrivée du chariot (24, 30) au niveau, respectivement, de la zone de chauffage (954), de la zone de chargement, et de la zone d'extraction.

13. Le dispositif de connexion de tubes stériles (10) de la revendication 12, dans lequel, sur un cycle d'utilisation du dispositif (10), la carte de circuit de contrôle électrique (17) est programmée pour contrôler la rotation du moteur (27) pour déplacer le chariot (24, 30) depuis la position de chauffage vers l'arrière jusqu'à la position de chargement pour charger la plaquette (95) sur le chariot (24, 30), depuis la position de chargement vers l'avant jusqu'à la position de chauffage pour chauffer la plaquette (95), depuis la position de chauffage vers l'avant jusqu'à la position d'extraction pour découper et joindre la première conduite (131) et la seconde conduite (131, 132), et depuis la position d'extraction vers l'arrière jusqu'à la position de chauffage pour retirer la plaquette (95) du chariot (24, 30).

14. Une plaquette de découpe (95) destinée à être utilisée avec le dispositif de connexion de tubes stériles (10) de l'une des revendications 1 à 13, dans laquelle la plaquette de découpe (95) comprend une zone de travail de plaquette (953) avec une unique couche d'un matériau métallique prévue pour être chauffée à la température prédéterminée pour effectuer une découpe au travers de la première conduite (131) et de la seconde conduite (131, 132), et une zone de chauffage de plaquette (954) comprenant des couches multiples de matériaux métalliques prévues pour recevoir de la chaleur provenant des éléments chauffants (611, 911) et pour délivrer la chaleur à la zone de travail de plaquette (953).

15. La plaquette de découpe (95) de la revendication 14, dans laquelle la zone de chauffage de plaquette (954) comprend deux couches du matériau métallique de la zone de travail de plaquette (953), les deux couches du matériau métallique étant reliées par une pliure, et/ou, en dépendance de la revendication 3, comprenant en outre une encoche de verrouillage de plaquette (956) destinée à venir en prise avec le verrou de plaquette (315) pour assujettir la plaquette (95) dans la fente support de plaquette (301) et empêcher un mouvement vers l'arrière de la plaquette (95).
